# EUROPEAN PATENT APPLICATION

(11) **EP 4 520 317 A1**
(43) Date of publication of application: **12.03.2025**
(21) Application number: 23195718.4
(22) Date of filing: 06.09.2023
(51) Int. Cl.: A61K 9/00, A61K 9/70

(54) **TRANSDERMAL THERAPEUTIC SYSTEM OF LUMATEPERONE**

(71) Applicant: Luye Pharma Switzerland AG, 4051 Basel (CH)
(72) Inventor: WAUER, Gabriel, 83727 Schliersee (DE); HENZEL, Claudia, 80803 Muenchen (DE); SCHURAD, Bjoern, 80807 Muenchen (DE); BENDA, Christian, 83730 Fischbachau (DE); STIEHLE, Andrea, 83714 Miesbach (DE)
(74) Representative: Kraus & Lederer PartGmbB

(57) **Abstract**

The present invention concerns a transdermal therapeutic system for administering the active ingredient lumateperone comprising a backing layer and at least one active ingredient containing layer, comprising the active ingredient in free base or salt form embedded in a polymer matrix, wherein the polymer matrix comprises a polymer selected from the group consisting of (1) a polyisobutene, (2) a styrene-butadienestyrene copolymer, (3) a silicone-acrylate hybrid polymer, (4) a styrene-isoprene-styrene copolymer, and mixtures thereof. The transdermal therapeutic system may further comprise an adhesive layer, a membrane layer, and a release liner.

## Description

### Technical field

The present invention concerns a transdermal therapeutic system for the administration of lumateperone.

### Background

Lumateperone, i.e. 1 -(4-fluoro-phenyl)-4-((6bR, 10aS)-3-methyl-2,3,6b,9,10,10a-hexahydro-1H,7H-pyrido[3',4':4,5]pyrrolo[1,2,3-de]quinoxalin-8-yl)-butan-1-one, also known as "ITI-007", is a potent 5-HT_{2A} receptor ligand with strong affinity for dopamine (DA)-D2 receptors and the serotonin transporter (SERT). Lumateperone has the following structure:

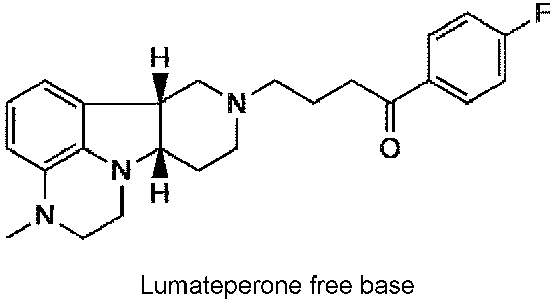

Lumateperone, analogues thereof, salts thereof, and methods for treatment comprising such compounds and methods of manufacturing such compounds, have been disclosed in various patent application documents, including e.g., WO 2020/047241 A1 and the further patent documents mentioned in paragraph [0003] of WO 2020/047241 A1 (in the following referred to as WO'241).

The drug has been approved for schizophrenia in December 2019 and for bipolar depression type I and II in December 2021 in the United States. The commercially available preparation of lumateperone is marketed under the trade name Caplyta^{®}. This product only allows for a once-daily administration in the form of oral capsules with dosages of 10.5 mg, 21 mg or 42 mg lumateperone per capsule. The active ingredient is used in form of its tosylate salt, wherein 60 mg (30 mg or 15 mg) per capsule correspond to 42 mg (21 mg or 10.5 mg) of the lumateperone free base.

The oral bioavailability of lumateperone is only about 4.4 %. Moreover, lumateperone has a strong first-pass metabolism in the liver. This makes the provision of alternative administration forms desirable.

Transdermal therapeutic systems (abbreviated herein as: TDS) is a well-known alternative to other dosage forms, such as oral dosage forms. Transdermal therapeutic systems are often associated with several advantages, including e.g. a higher bioavailability achieved via the transdermal administration, which allows to reduce the drug amount in the formulation; a better patient compliance achieved with a multi-day patch instead of the once daily oral administration; a circumvention of the first-pass; a reduction of plasma level fluctuations.

A transdermal therapeutic system of lumateperone is described in WO'241. The TDS according to WO'241 comprises lumateperone in free base or in salt form, an adhesive polymer, and optionally further excipients including permeation enhancing excipients and antioxidants. Permeation enhancers and certain combinations thereof are used to enhance permeation and the *in vivo* drug delivery. Antioxidants are said to improve the chemical stability of the formulations by preventing oxidative chemical degradation of the active ingredient.

Apart from the permeation properties (achieved with certain permeation enhancers and combinations thereof) and the chemical stability (in the short term), WO'241 fails to address certain further characteristics that are desirable for transdermal therapeutic systems.

While WO'241 generally discloses the use of acrylate adhesives, silicone adhesives and their blends. However, WO'241 contains no hint to use other polymer adhesives.

For example, WO'241 does not address at all the need for a high adhesiveness. Achieving a high adhesiveness is required for the provision of a patch that can be used for one day and especially important if use is foreseen for several days.

Furthermore, WO'241 does not address the need of providing a physically stable transdermal therapeutic system, i.e. the inhibition of crystallization of the active ingredient during storage. Crystallization of the active ingredient from the TDS is problematic because, besides the adverse effect on therapeutic efficacy and appearance of the matrix, it can also adversely affect in vitro drug release, adhesive strength, tack and shear of the TDS.

While according to WO'241, the drug formulations disclosed therein are chemically stable during storage at room temperature in the short term (i.e. up to two months), the need for chemical stability of the systems at the long-term conditions and accelerated conditions over a longer time period (i.e. for several months) has not been addressed in WO'241.

WO'241 moreover does not put any focus on the relation between drug loading and matrix weight. This is an important aspect to be considered for achieving a constant permeation over several days.

Finally, the use of permeation enhancers, which seem to be necessary in the systems according to WO'241 in order to achieve sufficient permeation, are often associated with disadvantages, e.g. that they cause skin irritation. They also complicate the preparation of the transdermal therapeutic system as they are often volatile on drying, sweat out in a lipophilic matrix, and adversely reduce tack and cohesion. From a regulatory point of view, enhancers make the development more difficult and expensive because they must be quantified, controlled, and their impact on the *in vivo* performance must be demonstrated. Specific requirements for enhancers are outlined in the "Guideline on excipients in the dossier for application for marketing authorisation of a medicinal product" (EMEA/CPMP/CVMP/QWP/396951/2006). In view of these disadvantages of using permeation enhancers, it would be desirable to provide a transdermal therapeutic system of lumateperone that has advantageous permeation properties without the need of using an enhancer.

In conclusion, there is still a need for further or improved transdermal therapeutic systems of lumateperone.

It is therefore the object of the present invention to provide a physically and chemically stable transdermal therapeutic systems of lumateperone showing a sufficiently high and substantially constant permeation as well as a high adhesiveness over preferably several days, such as e.g. at least 2 or 3 days, which allows the use of the transdermal therapeutic system as a multiday patch. Ideally, such transdermal therapeutic systems do not require the use of a permeation enhancer.

### Brief Summary

The present invention attempts to solve this object by the following aspects.

In a first aspect, the present invention provides a transdermal therapeutic system for administering the active ingredient lumateperone through the skin of a patient comprising the following layers:
a) a backing layer and
b) at least one active ingredient containing layer, comprising the active ingredient in free base or salt form embedded in a polymer matrix,
characterized in that
the polymer matrix comprises a polymer selected from the group consisting of (1) a polyisobutene, (2) a styrene-butadiene-styrene copolymer, (3) a silicone-acrylate hybrid polymer, (4) a styrene-isoprene-styrene copolymer, and mixtures thereof.

It has been found that the transdermal therapeutic systems according to the present invention have a sufficiently high and substantially constant permeation, preferably without the use of a permeation enhancer.

A "constant permeation" as used herein means that the permeation rate, expressed in amount permeated per unit of time and unit surface area, is not significantly changed over a prolonged time interval so that the cumulative permeation profile has a substantially linear course. However, in particular in the initial phase a so-called period of lag time can occur, wherein the permeation has not the constant permeation rate. Moreover, the permeation rate may be reduced at very late time points due to a very strong discharge of the system. The permeation can be determined by permeation tests *per* se known to the skilled person, for example by the *in vitro* permeation test according to NovoCell Schönbach according to OECD (2004) Test Guideline 428 "Skin absorption: In vitro Method & Series on testing and assessment", No. 28 "Guidance document for the conduct of skin absorption studies".

Moreover, it has been found that the transdermal therapeutic systems according to the present invention have a high adhesiveness suitable for at least one day, such as e.g. 2 or 3 days, which allows the use of the transdermal therapeutic systems as a multiday patch.

Moreover, the transdermal therapeutic systems of the present invention are physically and chemically stable during storage.

In a second aspect, the present invention provides a transdermal therapeutic system in accordance with the first aspect, wherein the system further comprises
c) an adhesive layer on the active ingredient containing layer,
wherein the adhesive layer comprises at least one adhesive polymer,
wherein the adhesive layer is initially free of the active ingredient, and
wherein the adhesive layer is the skin-contacting layer of the system.

"Initially free of active ingredient" means that the adhesive layer does not contain any active ingredient before coming into contact with the active ingredient layer. Once laminated, a certain amount of active ingredient diffuses from the active-ingredient layer into the adhesive layer.

It has been found that by using an adhesive layer in the transdermal therapeutics system in accordance with the second aspect of the present invention, the time period wherein permeation is constant can be increased, e.g. by 1 or 2 days, e.g. from 2 days to 3 days. Moreover, the adhesiveness of the transdermal therapeutic system can be increased. This prolongs the time period the transdermal therapeutic system can be used compared to a system which does not comprise an adhesive layer, e.g. by 1 or 2 days.

In a third aspect, the present invention provides a transdermal therapeutic system in accordance with the second aspect, wherein the system further comprises
d) a membrane layer between the active ingredient containing layer and the adhesive layer,
wherein the membrane layer comprises a membrane polymer that is permeable for the active ingredient and controls the release of the active ingredient.

It has been found that by using a membrane layer between the active ingredient containing layer and the adhesive layer in accordance with the third aspect of the present invention, the time period, wherein permeation is constant, can be increased, e.g. by 1 day to up to 3 days, compared to a system, which does not comprise a membrane layer.

In a further aspect, the present invention provides a method for preparing a transdermal therapeutic system in accordance with the present invention, comprising the following steps:
(a) preparing an active ingredient containing layer composition,
(b) coating the composition prepared in step (a) onto a release liner (i),
(c) drying the component prepared in step (b), and
(d) laminating a backing layer onto the active ingredient containing layer of the component prepared in step (c);
optionally comprising the following steps in addition to steps (a)-(d):
(e) preparing an adhesive layer composition,
(f) coating the composition prepared in step (e) onto a release liner (ii),
(g) drying the component prepared in step (f),
(h) removing the release liner (i) from the component prepared in step (d),
(i) laminating the active ingredient containing layer of the component prepared in step (h) onto the adhesive layer of the component prepared in step (g), or *vice versa;*
or optionally comprising the following steps in addition to steps (a)-(d):
(j) laminating a membrane layer suitable to control the release of the active ingredient onto the active ingredient containing layer of the component prepared in accordance with step (h),
(k) performing steps (e) to (g),
(l) laminating the adhesive layer of the component prepared in step (k) onto the membrane layer of the component prepared in step (j), or *vice versa.*

In a further aspect, the present invention relates to the use of a polymer selected from the group consisting of (1) a polyisobutene, (2) a styrene-butadiene-styrene copolymer, (3) a silicone-acrylate hybrid polymer, (4) a styrene-isoprene-styrene copolymer, and mixtures thereof for stabilizing lumateperone free base in a transdermal therapeutic system, or reducing degradation of lumateperone free base in a transdermal therapeutic system, preferably the transdermal therapeutic system of the present invention.

In a further aspect, the present invention relates to the used of an antioxidant, preferably ascorbic acid or tocopherol, in the transdermal therapeutic system of the present invention for reducing, preferably inhibiting the formation of N-nitrosamines in the system.

In a further aspect, the transdermal therapeutic system of the present invention is provided for use in a method for treating a disease selected from the group consisting of major depressive disorder (MDD) and adjunctive mixed features associated therewith, bipolar depression I or II and schizophrenia.

In a further aspect of the present invention, lumateperone is provided for use in a method for treating a disease selected from the group consisting of major depressive disorder (MDD) and adjunctive mixed features associated therewith, bipolar depression I or II and schizophrenia, wherein lumateperone is administered in free base or salt form through the skin of a patient by means of the transdermal therapeutic system of the present invention.

The above-mentioned various aspects are to be understood that they can be freely combined with each other unless otherwise indicated.

### Description of the Figures

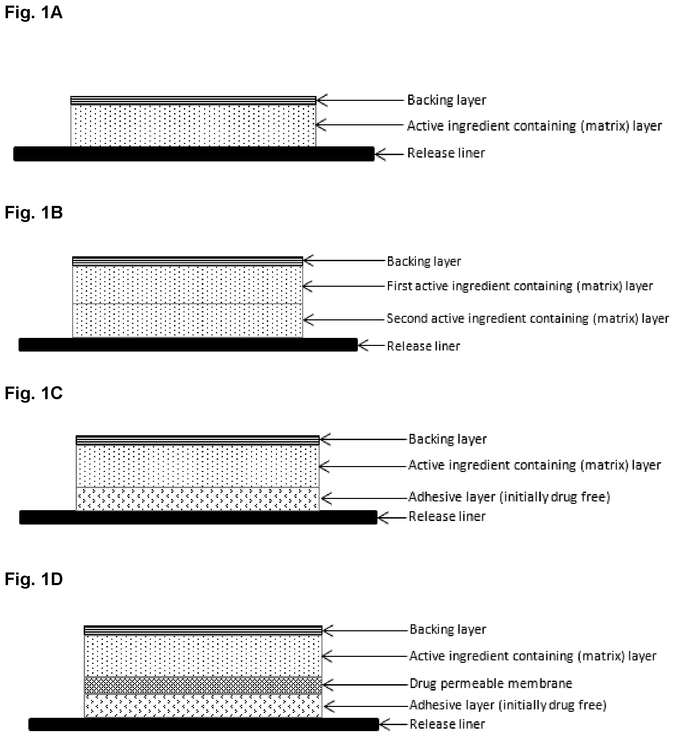

**Fig. 1** is a cross-sectional view of the TDS according to the present invention according to its various aspects. The TDS according to **Fig. 1A** comprises a backing layer, and active ingredient containing layer, and a release liner in accordance with the first aspect of the invention. The TDS according to **Fig. 1B** comprises a backing layer, a first active ingredient containing layer, a second active ingredient containing layer, and a release liner, also in accordance with the first aspect of the invention. The TDS according to **Fig 1C** comprises a backing layer, an active ingredient containing layer, an adhesive layer, and a release liner in accordance with the second aspect of the invention. The TDS according to **Fig 1D** comprises a backing layer, an active ingredient containing layer, a membrane layer, an adhesive layer, and a release liner in accordance with the third aspect of the invention.
**Fig. 2A-2D** show the adhesive strength measured for various samples prepared in respective Examples 1.1 to 1.4 after storage at 40°C/75% RH compared to prior art patches Neupro^{®} (1 day patch comprising rotigotine base and BIO-PSA Q7-4301 & 4201), FNT (3 days patch comprising fentanyl base and Duro-Tak^{®} 87-4098) and FTA (3 days patch comprising fentanyl base and Duro-Tak^{®} 387-2510). **Fig. 2E** shows the adhesive strength of various samples prepared in Example 1.2 with and without enhancer.
**Fig. 3A- 3D** show the tack for various samples prepared in respective Examples 1.1 to 1.4 measured after storage at 40°C/75% RH compared to prior art patch Neupro^{®}. **Fig. 3E** shows the tack for various samples prepared in Example 1.2 with and without enhancer.
Fig. 4A- 4D show the separation force needed to remove the release liner, measured for various samples prepared in respective Example 1.1 to 1.4 after storage at 40°C/75% RH compared to prior art patch FTA. **Fig. 4E** shows the separation force needed to remove the release liner for various samples prepared in Example 1.2 with and without enhancer.
Fig. 5A-D show the cumulatively permeated amount of lumateperone [µg/cm²] for various samples prepared in respective Examples 1.1 to 1.4 on human skin model (HSE) over a period 96 hours.
**Fig.** 6 shows the cumulatively permeated amount of lumateperone [µg/cm²] for various samples prepared in Example 1.2 on human skin model (HSE) over a period of 96 hours.
**Fig. 7A** shows the chemical stability of samples prepared in Examples 1.1 and 1.3 after storage at 40°C/75% RH for 6 months. **Fig. 7B** shows the chemical stability of samples prepared in Example 1.2 after storage at 40°C/75% RH for 6 months

### Detailed Description

In the following, the present invention will be described in further detail and, unless it is explicitly stated otherwise, the explanations on the individual features refer to all of the aspects of the present invention and can be freely combined with one another.

### Definitions

The transdermal therapeutic system ("TDS") of the present invention can also be designated as a patch. The TDS of the present invention is for application on the skin of a patient in need thereof, i.e. a patient in need of lumateperone treatment.

The "active ingredient" within the meaning of the present invention is lumateperone, i.e. 1-(4-fluoro-phenyl)-4-((6bR,10aS)-3-methyl-2,3,6b,9,10,10a-hexahydro-1H,7H-pyrido[3',4':4,5]pyrrolo[1,2,3-de]quinoxalin-8-yl)-butan-1-one. The active ingredient is used in its free base form or in salt form in the TDS of the present invention. "In salt form" means in pharmaceutically acceptable salt form, wherein the salt form can be amorphous or crystalline polymorph. The salt form of lumateperone can be selected from the group consisting of a tosylate, oxalate, cyclamate, 4-aminosilicylate, or hydrochloride salt form, with the tosylate being the preferred salt form. However, in the present invention, it is preferred that the free base form of lumateperone is used. This applies to all aspects and embodiments of the present invention.

A "polymer matrix" is a solid or semi-solid composition having a three-dimensional structure which comprises a polymer or a mixture of polymers. In the present invention, the polymer matrix comprises an acrylate polymer or copolymer containing free hydroxy groups. The polymer matrix is also referred to as polymer skeleton since the three-dimensional skeleton structure is as a rule provided by the polymer or mixture of polymers. Preferably, the active ingredient is evenly distributed in the polymer matrix.

As described herein, the term "a" refers to one or more elements. For example, the term "an acrylate polymer" refers to one or more acrylate polymers.

Percentages, unless otherwise indicated, refer to weight percentages, abbreviated "wt.-%". Weight percentage amounts, unless otherwise indicated, are given in relation to a dried product, for example a dried active ingredient containing layer or a dried adhesive layer.

In the TDS according to the invention, the active ingredient is chemically sufficiently stable. "Sufficiently stable" means that degradation products of the active ingredient after 1 month of storage at 40°C and 75% relative air humidity (RH) in total are preferably not more than about 1% by weight, more preferably not more than about 0.5% by weight, based on the desired content of active ingredient in the system. Preferably, the total content of the degradation products after 3 months of storage at 40°C and 75% relative air humidity is less than about 2% by weight, more preferably less than about 0.6% by weight. It is also preferred that the total content of the degradation products after 6 months of storage at 40°C and 75% relative air humidity is less than about 4% by weight, more preferably less than 3% by weight. The information on "wt.-%" of degradation products always refer to the amount of active ingredient in the formulation, unless stated otherwise.

In the TDS according to the present invention, the active ingredient is also physically stable. "Physically stable" means that crystallization of the active ingredient is inhibited during storage at room temperature for at least 12 months, or at 40°C and 75% relative air humidity for at least 6 months, preferably 12 months. Preferably, "physically stable" also means that the adhesive strength, tack, and separation force stability are substantially maintained during storage at room temperature for at least 12 months, or at 40°C and 75% relative air humidity for at least 6 months, preferably 12 months. The term "substantially maintained" refers to a change of no more than ± 10%, no more than ± 5%, or no more than ± 2.5%.

The application period of a TDS according to the invention is at least one day, preferably at least 2 days, such as e.g. 2 to 3 days, more preferably at least 3 days, such as e.g. 3 to 4 days.

Although it is possible to use a permeation enhancer in the TDS of the present invention (i.e. in the active ingredient containing layer and/or the adhesive layer), it is preferred that the TDS of the present invention does not comprise a permeation enhancer. In particular, the TDS of the present invention does not comprise the permeation enhancers used in WO'241, which include fatty acid esters (e.g. lauryl lactate, isopropyl myristate, oleyl oleate, methyl laurate, isopropyl palmitate, ethyl oleate), fatty alcohols (dodecanol, octyldecanol, lauryl alcohol, alcohols (e.g. propylene glycol), amine oxides (e.g. dimethyldodecyl amine oxide, myristamine oxide), carboxylic acids (e.g. an alpha-hydroxy acid, e.g. lactic acid), caprates (e.g. methyl caprate, propylene glycol dicaprate/dicaprylate), and combinations thereof such as e.g. lauryl lactate/propylene glycol.

### Structure of the TDS

The TDS of the present invention comprises several layers.

The backing layer is situated on the back side of the TDS.

The active ingredient containing layer is situated onto the side of the backing layer which is in direction to the skin after TDS application. In the first aspect of the present invention, the active ingredient containing layer is the skin-contacting layer, which is protected by a release liner during storage. The first aspect of the present invention thus concerns a TDS comprising the following layers in the following order (cf. **Fig. 1A**):
1. a backing layer
2. a first active ingredient containing layer
3. a release liner.

In one embodiment, the TDS of the present invention comprises two active ingredient containing layers laminated to each other. In that case, a TDS in accordance with the first aspect of the present invention comprising the following layers in the following order (**Fig. 1B**).
1. a backing layer
2. a first active ingredient containing layer
3. a second active ingredient containing layer
4. a release liner.

In the second aspect of the present invention, an adhesive layer is deposited to the active ingredient containing layer that faces the human skin in use. In this second aspect of the present invention, the adhesive layer is the skin-contacting layer, which is protected by a release liner during storage. The second aspect of the present invention thus concerns a TDS comprising the following layers in the following order (**Fig. 1C**).
1. a backing layer
2. an active ingredient containing layer
3. an adhesive layer
4. a release liner.

In the third aspect of the present invention, the TDS comprises a membrane layer deposited between the active ingredient containing layer and the adhesive layer. The third aspect of the present invention thus concerns a TDS comprising the following layers in the following order (**Fig. 1D**):
1. a backing layer
2. an active ingredient containing layer
3. a membrane layer
4. an adhesive layer
5. a release liner

Before the TDS is used, the release liner is deposited to the active ingredient containing layer (first aspect) and the adhesive layer (second and third aspect of the invention), respectively, and protects those layers before use of the TDS. The release liner is removed right before the use of the TDS.

In one embodiment, the area of the backing layer of the TDS according to the invention corresponds at least to the area of the active ingredient containing layer or the adhesive layer, respectively.

### Active ingredient containing layer

The TDS according to the present invention comprises at least one active ingredient containing layer. Typically, this means that the TDS of the present invention contains one active ingredient containing layer. However, it is also possible that the TDS of the present invention contains two active ingredient containing layers, in particular if the weight of the active ingredient containing layer exceeds 100 g/m². In that case, two active ingredient containing layers may be used instead of one. The two layers are typically identical in terms of their composition and size and are laminated to one another to achieve a weight of 100 g/m² and more, such as e.g. 100 to 150 g/m². For example, if a matrix weight of 130 g/m² is attempted, two active ingredient containing layers with a matrix weight of 75 g/m² each can be used.

The active ingredient containing layer of the TDS according to the invention contains the active ingredient lumateperone in free base form or in salt form, embedded in a polymer matrix.

Preferably, the active ingredient containing layer of the TDS according to the present invention contains 5 to 20 wt.-%, more preferably 10 to 15 wt.-%, particularly preferably 11 to 13 wt.-% of the active ingredient lumateperone.

Preferably, the active ingredient containing layer of the TDS according to the present invention contains 65 to 95 wt.-%, more preferably 70 to 90 wt.-% or 75 to 85 wt.-%, particularly preferably 80 to 85 wt.-% of the polymer matrix.

The polymer matrix comprises a polymer selected from the group consisting of (1) a polyisobutene, (2) a styrene-butadiene-styrene copolymer, (3) a silicone-acrylate hybrid polymer, (4) a styrene-isoprene-styrene copolymer, and mixtures thereof.

### (1) Polyisobutene

Polyisobutene (PIB), also referred to as polyisobutylene, is commercially available.

Examples for polyisobutenes (also called polyisobutylenes) suitable for use in the present invention include the commercial products Duro-Tak^{®} 87-6900, Duro-Tak^{®} 87-626A, and Duro-Tak^{®} 87-625A or mixtures thereof. In an embodiment, the polymer matrix contains only one polyisobutene. In a preferred embodiment, the polymer matrix comprises a mixture of two or more polyisobutenes. In an embodiment, the polymer matrix comprises at least two polyisobutenes. In an embodiment, the polymer matrix comprises two polyisobutenes, PIB A and PIB B, in a wt.-ratio of PIB A to PIB B of 1:10 to 10:1, or of 1.3 to 3:1.

Preferred examples for suitable polyisobutenes for use as adhesive polymer in the adhesive layer of the TDS of the present invention are the products Duro-Tak^{®} 87-626A and Duro-Tak^{®} 87-6900. Those products are preferably used as a 1:1 mixture. Those two products have a different molecular weight.

It has further been found that the adhesive strength of the active ingredient containing layer based on polyisobutene can be increased by adding polybutene, which is a plasticizer, such as e.g. the products Indopol^{®} H-1900 and H-18000. Indopol^{®} H-1900 is a polybutene with an average molecular weight M*_{N}* of about 2,500 g/mol. Indopol^{®} H-18000 is a polybutene with an average molecular weight M*_{N}* of about 6,000 g/mol. The "average molecular weight M*_{N}*" is the number-average molar mass and can be determined according to American Standard ASTM D3536-91 or ASTM D5296-05. Polyisobutene and polybutene are preferably used in a weight ratio of 4:1 to 1:2, more preferably 3:1 to 1:2. For example, polyisobutene and polybutene can be present in a weight ratio of about 1:1.

It has been found in the present invention that when using a polymer matrix based on (1) a polyisobutene, (2) a styrene-butadiene-styrene copolymer, (3) a silicone-acrylate hybrid polymer, (4) a styrene-isoprene-styrene copolymer, and mixtures thereof, sufficient high permeation could be achieved leading to fluxes high enough for a sufficient small patch size. This could be achieved without use of skin permeation enhancers. Moreover, (1) a polyisobutene, (2) a styrene-butadiene-styrene copolymer, (3) a silicone-acrylate hybrid polymer, (4) a styrene-isoprene-styrene copolymer, and mixtures thereof show promising high adhesiveness in form of adhesive strength and tack compared to marketed one day and 3 day patches. Regarding chemical stability the four polymer classes show sufficient or even good stability of the active ingredient lumateperone free base contained in the active ingredient containing layer compared to acrylate polymers of the prior art.

### (2) Styrene-butadiene-styrene copolymer

A styrene-butadiene-styrene copolymer, also referred to as styrenic rubber polymer, is also suitable as polymer for the polymer matrix. It is preferred that this copolymer is a block copolymer.

Examples of suitable silicone adhesives include Duro-Tak^{®} 87-6911 (containing petroleum-based hydrocarbon resin) andDuro-Tak^{®} 87-6173 (containing resin from hydrogenated rosin glycerol ester). In a particularly preferred embodiment, the styrene-butadiene-styrene copolymer is the product Duro-Tak^{®} 87-6911, which is a mixture of styrene-butadiene-styrene and styrene-butadiene block copolymers and tackifier (resin) consisting of petroleum-based hydrocarbon resin.

### (3) Silicone-acrylate hybrid polymer

The silicone-acrylate hybrid polymer comprises a polymerized hybrid species that includes silicone-based sub-species and acrylate-based sub-species that have been polymerized together. The silicone-acrylate hybrid polymer thus comprises a silicone domain and an acrylic domain. Preferably, the silicone-acrylate hybrid polymer is a silicone-acrylate hybrid polymer pressure-sensitive adhesive.

Preferably, the weight ratio of silicone to acrylate in the silicone-acrylate hybrid polymer is from 5:95 to 95:5, or from 20:80 to 80:20, more preferably from 40:60 to 60:40, and most preferably the ratio of silicone to acrylate is about 50:50.

Examples of suitable silicone-acrylate hybrid polymersinclude BIO-PSA 7-6302 (high tack; in ethyl acetate), and BIO-PSA 7-6102 (low tack; in ethyl acetate), as well as BIO-PSA 7-6301 (high tack; in n-heptane) and BIO-PSA 7-6101 (low tack; in n-heptane) as well as Duro-Tak^{®} SH 87-500.

Preferably, silicone-acrylate hybrid polymers are used which use ethyl acetate as solvent wherein the active ingredient in its free base form is soluble. The solid content in such commercially available products is usually between 30% and 70%.

### (4) Styrene-isoprene-styrene copolymer

Styrene-isoprene-styrene copolymer are preferably block copolymers.

Examples of styrene-isoprene-styrene copolymers include e.g. Styrene, Isoprene and Styrene Block Copolymer from the company JSR Corporation (Japan), SIS 5002, SIS 5229.

In one embodiment, the active ingredient containing layer consists of active ingredient and polymer matrix.

Optionally, further excipients known in the prior art are contained in the active ingredient containing layer besides the active ingredient and the matrix polymer.

Preferably, such further excipients are selected from the group consisting of antioxidants, permeation enhancers, plasticizers, tackifiers, crystallization inhibitors, and cohesion increasing substances. Further, substances may be added that trigger the *in situ* release of lumateperone free base in case the active ingredient is used in a salt form.

The excipients are typically used in an amount of up to 75 wt.-% in the active ingredient containing layer, such as e.g. 0.05 to 75 wt.-%, 0.1 to 70 wt.-%, 0.5 to 65 wt.-%, 1 to 60 wt.-%, 1 to 55 wt.-%, 1 to 50 wt.-%, 1 to 45 wt.-%, 2 to 40 wt.-%, 2.5 to 35 wt.-%, 2.5 to 10 wt.-%, 5 to 10 wt.-%, or 10 to 15 wt.-%, based on the overall weight of the (dried) active ingredient containing layer.

Suitable antioxidants for use in the active ingredient containing layer of the system of the present invention include tocopherol, butylated hydroxytoluene (BHT), propyl gallate (OPG), N,N'-bis[3-propanamid] Irganox^{®}, ris(2,4-di-tert-butylphenyl) phosphite Irgafos^{®}, ascorbic acid, ascorbyl palmitate, and combinations thereof. The antioxidant is typically contained in the active ingredient containing layer in an amount of 0.05 to 2 wt.-%, preferably 0.05 to 1.75 wt.-%, more preferably 0.05 to 1.5 wt.-%, even more preferably 0.05 to 1.1 wt.-%, based on the overall weight of the active ingredient containing layer. Tocopherol or/and ascorbyl palmitate are preferred antioxidants to be used in the active ingredient containing layer. Antioxidants have the effect that they improve the chemical stability of the active ingredient in particular during storage of the TDS by preventing oxidative chemical degradation. They may also contribute to a inhibition of N-Nitrosoamine formation in the drug product.

It has also been found by the present inventors that the use of an antioxidant in the TDS of the present invention, in particular in the active ingredient containing layer of the TDS of the present invention, advantageously reduces, preferably inhibits the formation of N-nitrosamines in particular during storage of the transdermal therapeutic system due to degradation of the active ingredient. The skilled person understands the term N-nitrosamines and what compounds these are. N-nitrosamines are carcinogenic compounds that are accepted by regulatory authorities only in extremely small amounts in pharmaceutical products including transdermal therapeutic systems. N-nitrosamines may form in active ingredients that have a secondary or tertiary amine function (such as lumateperone) under certain conditions that promote formation of nitrosamines (e.g. high temperatures during storage in the finished product). It has been found by the present inventors that antioxidants, in particular ascorbic acid and tocopherol, reduce, preferably avoid the formation of N-nitrosamines in the TDS of the present invention by stabilizing the active ingredient in particular during storage.

Plasticizers (softeners) suitable for use in the active ingredient containing layer include Polybutenes, fatty acid esters such as decanoyl and octanoyl glycerides or mixtures thereof (such as the product Miglyol^{®} 812), and mixtures of hydrocarbons such as paraffin. A plasticizer is typically contained in the active ingredient containing layer in an amount of 1 to 5 wt.-%, such as e.g. 2.5 wt.-%, based on the overall weight of the (dried) active ingredient containing layer. In an embodiment, the TDS of the present invention does not contain a plasticizer. In an embodiment, the active ingredient containing layer comprises 1 to 60 wt.-% of a plasticizer. In particular when the polymer matrix comprises polyisobutene, it is preferred that the active ingredient containing layer comprises 1 to 60 wt.-%, or 20 to 50 wt.-% of a plasticizer.

Permeation enhancers suitable for use in the present invention include those disclosed in WO'241, i.e. fatty acid esters (e.g. lauryl lactate, isopropyl myristate, oleyl oleate, methyl laurate, isopropyl palmitate, ethyl oleate), fatty alcohols (dodecanol, octyldecanol, lauryl alcohol, alcohols (e.g. propylene glycol), amine oxides (e.g. dimethyldodecyl amine oxide, myristamine oxide), carboxylic acids (e.g. an alpha-hydroxy acid, e.g. lactic acid), caprates (e.g. methyl caprate, propylene glycol dicaprate/dicaprylate), and combinations thereof such as e.g. lauryl lactate/propylene glycol. A permeation enhancer or a combination of permeation enhancers is typically contained in the active ingredient containing layer in an amount of 1 to 10 wt.-%, based on the overall weight of the active ingredient containing layer. However, in the present invention, it is preferred that the active ingredient containing layer does not contain a permeation enhancer. It is particularly preferred that the active ingredient containing layer does not contain the permeation enhancers used in the transdermal therapeutic system disclosed in WO'241.

Cohesion increasing substances suitable for use in the active ingredient containing layer include cationic copolymer from dimethylaminoethylmethacrylate, butylmethacrylate and methyl methacrylate, such as e.g. Eudragit^{®} E100, butyl methacrylate and methylmethacrylate copolymer Plastoid^{®} B, polyvinylpyrrolidone, polyvinylacetate.

Cohesion increasing substances suitable for use in the active ingredient containing layer also include cationic crosslinkers containing cationic aluminium (such as e.g. aluminium actylacetonate) or titanium (such as e.g. titanium butoxide).

Crystallization inhibitors prevent crystallization of the active ingredient in the active ingredient containing layer. Suitable crystallization inhibitors for use in the active ingredient containing layer include polyvinyl pyrrolidone (PVP), polyvinylacetate, and cellulose derivates.

In addition, the active ingredient containing layer may contain a tackifier. Tackifier can preferably be selected from resin from hydrogenated rosin glycerol ester, petroleum-based hydrocarbon resin, polybutene, alicyclic hydrogenated hydrocarbon resin (Arkon^{®} P-100), and mixtures thereof. The content of tackifier in the active ingredient layer may be 0 to 80% by weight, preferably 25 to 75% by weight, in particular 40 to 70% by weight, e.g. about 50% by weight, in each case based on the dried total weight of the active ingredient layer.

If the active ingredient lumateperone is used in salt form, e.g. as tosylate salt, the active ingredient containing layer may comprise further substances that trigger the *in situ* release of lumateperone free base. These substances include inorganic or organic bases, such as alkali metal or alkaline earth metal carbonates, bicarbonates, hydroxides, oxides, and diisopropanolamine.

It is particularly preferred that the active ingredient containing layer comprises a styrene-butadiene-styrene copolymer and a petroleum-based hydrocarbon resin (e.g. Duro-Tak^{®} 87-6911). In an even more preferred embodiment, the wt.-ratio of styrene butadiene-styrene copolymer to petroleum-based hydrocarbon resin is from 1:5 to 5:1, from 1:3 to 3:1, or from 1:2.5 to 2.5:1.

It is particularly preferred that the active ingredient containing layer comprises a silicon-acrylate hybrid polymer and an alicyclic hydrogenated hydrocarbon resin.

It is particularly preferred that the active ingredient containing layer comprises a polyisobutene and polybutene. In an even more preferred embodiment, the wt.-ratio of polyisobutene to polybutene is from 1:5 to 5:1, from 1:3 to 3:1, or from 1:2.5 to 2.5:1.

The absolute amount of the active ingredient in the active ingredient containing layer of the TDS according to the present invention depends on different factors, in particular the size of the TDS to be used, the matrix weight, and the active ingredient concentration in the active substance containing layer. Exemplary amounts of the active ingredient lumateperone (free base equivalent) to be used in the TDS of the present invention are in the range from 2 to 43.2 mg, e.g. 2 to 28.8 mg, 2 to 27 mg, 2 to 18 mg, 4.5 to 12.5 mg, 6.75 to 18.75 mg, 7.2 to 20 mg, 10.8 to 30 mg, 8 to 11 mg, 12 to 16.5 mg, or 12.8 to 17.6 mg, 29.2 to 26.4 mg. If a salt form of lumateperone is used, these amounts have to be adapted taking into consideration the differing molar weights of lumateperone free base and its salt forms, respectively.

The size of the active ingredient containing layer is typically in the range from 4 to 40 cm². In an embodiment, the size of the active ingredient containing layer is not larger than 36 cm² preferably not larger than 25 cm², more preferably not larger than 22 cm², most preferably not larger than 20 cm², such as e.g. 18, 16, 14, 12, 10, 8, or 6 cm².

The weight of the dried active ingredient containing layer ("matrix weight") is preferably in the range from 20 to 100 g/m², more preferred in the range of 30 to 90 g/m², and still more preferred in the range from 40 to 80 g/m². For a 1 day patch, the weight is typically in the range from 40 to 50 g/m². For a 1 to 2 days patch, the weight is typically in the range from 40 to 60 g/m². For a 2 to 3 days patch, the weight is typically in the range from 50 to 70 g/m². For a 3 to 4 days patch, the weight is typically in the range from 65 to 80 g/m².

Exemplary combinations that take into account the size of the transdermal therapeutic system, the drug loading, and the matrix weight are as follows (with API = active pharmaceutical ingredient = lumateperone free base):
4 cm² TDS, 10% API (2 mg), 50 g/m²
9 cm² TDS, 10% API (4.5 mg), 50 g/m²
16 cm² TDS, 10% API (8 mg), 50 g/m²
20 cm² TDS, 10% API (10 mg), 50 g/m²
22 cm² TDS, 10% API (11 mg), 50 g/m²
25 cm² TDS, 10% API (12.5 mg), 50 g/m²
36 cm² TDS, 10% API (18 mg), 50 g/m²
4 cm² TDS, 15% API (3 mg), 50 g/m²
9 cm² TDS, 15% API (6.75 mg), 50 g/m²
16 cm² TDS, 15% API (12 mg), 50 g/m²
20 cm² TDS, 15% API (15 mg), 50 g/m²
22 cm² TDS, 15% API (16.5 mg), 50 g/m²
25 cm² TDS, 15% API (18.75 mg), 50 g/m²
36 cm² TDS, 15% API (27 mg), 50 g/m²
4 cm² TDS, 10% API (3.2 mg), 80 g/m²
9 cm² TDS, 10% API (7.2 mg), 80 g/m²
16 cm² TDS, 10% API (12.8 mg), 80 g/m²
20 cm² TDS, 10% API (16 mg), 80 g/m²
22 cm² TDS, 10% API (17.6 mg), 80 g/m²
25 cm² TDS, 10% API (20 mg), 80 g/m²
36 cm² TDS, 10% API (28.8 mg), 80 g/m²
4 cm² TDS, 15% API (4.8 mg), 80 g/m²
9 cm² TDS, 15% API (10.8 mg), 80 g/m²
16 cm² TDS, 15% API (19.2 mg), 80 g/m²
20 cm² TDS, 15% API (24 mg), 80 g/m²
22 cm² TDS, 15% API (26.4 mg), 80 g/m²
25 cm² TDS, 15% API (30 mg), 80 g/m²
36 cm² TDS, 15% API (43.2 mg), 80 g/m².

### Adhesive layer

In a preferred embodiment of the present invention, the transdermal therapeutic system of the present invention further comprises an adhesive layer. In this embodiment, the adhesive layer is the skin-contacting layer of the system. The adhesive layer has the effect that it increases the adhesiveness of the system. Furthermore, the adhesive layer increases the duration of the permeation, i.e. it prolongs the time that the transdermal system can be used.

The adhesive layer comprises an adhesive polymer and is initially free of the active ingredient lumateperone. "Initially free", as already explained above, means that the adhesive layer is prepared without the active ingredient and that the adhesive layer is accordingly free of the active ingredient before the adhesive layer is laminated with the active ingredient containing layer. When the TDS is manufactured by laminating the active ingredient containing layer with the adhesive layer, a certain amount of the active ingredient is distributing into the initially active ingredient free adhesive layer by diffusion. The adhesive layer is then no longer free of the active ingredient. This is usual and does not affect the performance of the TDS.

The adhesive polymer used in the adhesive layer is preferably selected from the group consisting of acrylate polymers and co-polymers, styrene-butadiene-styrene (SBS) copolymers, and polyisobutylene, and mixtures thereof.

In a preferred embodiment, polyacrylate homopolymers, copolymers, and blockcopolymers on the basis of acrylic acid esters and/or methacrylic acid esters can be used as adhesive polymers. As monomers for the production of suitable polyacrylates in particular n-butyl acrylate, n-butyl methacrylate, ethyl acrylate, 2-ethylhexyl acrylate, ethyl methacrylate, methyl acrylate, methyl methacrylate, tert-butyl acrylate, sec-butyl acrylate, tert-butyl methacrylate, cyclohexyl methacrylate, 2-ethylhexyl methacrylate, isobornyl methacrylate, isobutyl methacrylate, isopropyl acrylate, isopropyl methacrylate and mixtures of these monomers are possible. These monomers are esters of the acrylic or methacrylic acid, respectively, that carry linear, branched or cyclic aliphatic C1-C12 substituents without other free functional groups. Also vinyl acetate can be used as a comonomer together with at least one of these monomers for the production of the polyacrylate.

In a more preferred embodiment, the acrylate polymers and co-polymers used as adhesive polymers comprise free hydroxy groups. Examples include the products Duro-Tak^{®} 87-2516 and 387-2516, which are acrylate-vinyl acetate copolymers containing free hydroxy-groups.

In another more preferred embodiment, the acrylate polymers and co-polymers used as adhesive polymers comprise free carboxylic acid groups. Examples include the products Duro-Tak^{®} 87-2051 and 387-2051, which are acrylate-vinyl acetate copolymers containing free carboxylic acid groups.

In another preferred embodiment, the adhesive polymer is a styrene-butadiene-styrene copolymer. In a particularly preferred embodiment, the styrene-butadiene-styrene copolymer is the product Duro-Tak^{®} 87-6911, which is a mixture of styrene-butadiene-styrene and styrene-butadiene block copolymers and tackifier(resin) consisting of petroleum-based hydrocarbon resin.

It has further been found that the adhesive strength of an adhesive layer based on polyisobutylene can be increased by adding polybutene, which is also an adhesive polymer, such as e.g. the products Indopol^{®} H-1900 and H-18000. Indopol^{®} H-1900 is a polybutene with an average molecular weight M*_{N}* of about 2,500 g/mol. Indopol^{®} H-18000 is a polybutene with an average molecular weight M*_{N}* of about 6,000 g/mol. The "average molecular weight M*_{N}*" is the number-average molar mass and can be determined according to American Standard ASTM D3536-91 or ASTM D5296-05. Polyisobutylene and polybutene are preferably used in a weight ratio of 4:1 to 1:2, more preferably 3:1 to 1:2. For example, polyisobutylene and polybutene can be present in a weight ratio of about 1:1.

Preferably, the adhesive layer of the TDS according to the present invention contains 80 to 100 wt.-% of the at least one adhesive polymer, such as e.g. 85 to 100 wt.-%. 90 to 100 wt.-%, 95 to 100 wt.-% or 97.5 to 100 wt.-%, based on the overall weight of the adhesive layer.

The adhesive layer preferably consists of the at least one adhesive polymer. Optionally, it further comprises one or more excipients selected from the group consisting of adhesiveness enhancers, plasticizers, cohesion increasing substances, antioxidants, which are typically contained in an amount of up to 20 wt.%, such as e.g. 15 wt.%, 10 wt.%, 5 wt.%, or 2.5 wt.-%, based on the overall weight of the adhesive layer.

Adhesiveness enhancers increase the adhesive strength of the adhesive layer. Surprisingly, it has been found that the adhesive strength of an adhesive layer based on acrylate polymers and co-polymers can be increased by adding Miglyol^{®} 812 in a small amount of e.g. 2 wt.-%, based on the overall weight of the adhesive layer. Miglyol^{®} 812 is typically used as a plasticier (softener), and not as an adhesiveness enhancer.

The weight of the (dried) adhesive layer is typically in the range from 10 to 50 g/m², preferably in the range from 20 to 40 g/m², such as e.g. 30 g/m².

### Membrane layer

In a third aspect of the present invention, a membrane layer between the active ingredient containing layer and the adhesive layer is used, wherein the membrane layer comprises a membrane polymer that is permeable for the active ingredient and controls the release of the active ingredient. The membrane layer enables the prolongation of the constant permeation time for several days. At the same time, however, permeation is reduced compared to a TDS without membrane layer.

Preferably, the membrane polymer consists of a polyolefine such as for example polypropylene (PP) (e.g., Celgard^{®} 2400), or of polyethylene (PE) (e.g., CoTran^{™} 9719 or CoTran^{™} 9720), or of polyethylene with a vinyl acetate proportion (EVA), such as a vinyl acetate proportion of e.g. 4.5 to 19% (e.g. CoTran^{™} 9707; CoTran^{™} 9702; CoTran^{™} 9728). Moreover, the membranes can have a porosity of up to 90% (e.g. Solupor^{®} 10P05A, Celgard^{®} 2400).

Porous membranes or coherent membranes may be used.

The porosity of the porous membranes can be up to about 90%. Examples of preferred porous membranes are:
Solupor^{®} 10P05A (polyethylene, porosity: 83%, thickness: 60 µm)
Celgard^{®} 2400 (polypropylene, porosity: 41%, thickness: 25 µm)

Typically, the membrane has a thickness of 0.01 and 0.15 mm. The preferred thickness of the membrane is 0.020 to 0.080 mm.

According to the invention, it is particularly preferred to use a coherent membrane substantially consisting of polyethylene with a thickness of about 40 to 50 µm (e.g. CoTran^{™} 9719), which enables prolongation of the constant permeation time for up to 3 days; or a porous membrane consisting of polypropylene with a thickness of about 20 to 40 µm (e.g. Celgard^{®} 2400), which enables prolongation of the constant permeation time for up to three days, but on the other hand maintaining a sufficiently high permeation to allow a small patch size of e.g. about 36 or, preferably, 20 cm² or smaller.

### Backing layer

The TDS of the present invention comprises a backing layer. Typically, the backing layer of the TDS according to the invention is occlusive, that is it is the outermost layer in the cross-section of the finished TDS which is furthest away from the skin contacting layer. The backing layer is an inert layer which is substantially free of the active ingredient and substantially impermeable for the active ingredient.

In a preferred embodiment, such backing layers consist of polyolefins, in particular polyethylene, or polyesters as well as polyurethanes. Also layers containing several different polymers arranged on top of each other can preferably be used. Suitable materials comprise polyolefin, cellophan, cellulose acetate, ethyl cellulose, vinyl acetate-vinyl chloride copolymers provided with plasticizers, ethylene-vinylacetate copolymers, polyethylene terephthalate, nylon, polyethylene, polypropylene, polyvinylidene chloride, ethylene-methacrylate copolymers, paper which can optionally be coated, textile tissue, aluminum foil, and polymer-metal composite materials. Polyester foils, such as polyethylene terephthalate foils, e.g. the product Hostaphan^{™} MN 19 are particularly preferred.

Especially preferred are composite foils of tan pigmented polyethylene, thermoplastic resin and aluminum vapor coated polyester, e.g. the product Scotchpack^{™} 9738 or lacquered metalized Hueck PET foils. Polymer-metal composite materials, such as e.g. the product Scotchpack^{™} 9738 or lacquered metalized Hueck PET foils, protect the active ingredient against light-induced degradation in particular during storage. Polymer-non-metal composite materials, e.g. Scotchpak^{™} 9723 or lacquered Hueck PET foils, can also be used in case the composition of the backing layer sufficiently inhibits light-induce degradation through e.g. pigments or excipients within a lacquer or outer layer.

As is common in the prior art, the thickness of the back layer may be for example 10 µm to 100 µm, for example about 40 µm (nominal thickness). This ensures flexibility and wearing comfort of the transdermal therapeutic system of the present invention.

### Release liner

In a preferred embodiment of the present invention, the skin-contacting layer of the TDS, i.e. the active ingredient containing layer (first aspect) or the adhesive layer (second and third aspect) is protected with a release liner. The release liner is deposited on the skin-contacting layer and is removed when the TDS is to be used.

Preferably, the release liner is prepared from polymeric material that optionally can also be metallized. Examples of preferably used materials are polyurethanes, polyvinylacetate, polyvinylidene chloride, polypropylene, polycarbonate, polystyrene, polyethylene, polyethylene terephthalate, polybutylene terephthalate as well as paper that is optionally surface coated with corresponding polymers. The release liner can be coated, e.g. fluoropolymer or non-fluoropolymer-coated, silicone- or fluorosilicone-coated on one or both sides, wherein siliconized release liners are particularly suitable for polyacrylate-based layers. Particularly preferred are coated polyester foils, such as the one-sided siliconized commercial products Primeliner^{™} 75 or 100 µm and Perlasic LF 75 µm (Loparex, NL and Perlen Converting AG, Switzerland) and the silicon-coated polyethylenetherepthalate (PET) (e.g. Loparex Primeliner^{™} 78HL), or the one-sided fluoropolymer-coated products such as e.g. Scotchpak^{™} 1022 (3M Drug delivery). Further suitable products are Scotchpak^{™} 9744 and Scotchpak^{™} 9709.

### Method for the preparation of the TDS according to the present invention

A further aspect of the present invention is to provide a method for the production of the TDS according to the invention. The method comprises the following steps:
(a) preparing an active ingredient containing layer composition,
(b) coating the composition prepared in step (a) onto a release liner (i) ,
(c) drying the component prepared in step (b), and
(d) laminating a backing layer onto the active ingredient layer of the component prepared in step (c);
optionally comprising the following steps in addition to steps (a)-(d):
(e) preparing an adhesive layer composition,
(f) coating the composition prepared in step (e) onto a release liner (ii),
(g) drying the component prepared in step (f),
(h) removing the release liner (i) from the component prepared in step (d),
(i) laminating the active ingredient containing layer of the component prepared in step (h) onto the adhesive layer of the component prepared in step (g), or *vice versa;*
or optionally comprising the following steps in addition to steps (a)-(d):
(j) laminating the membrane layer suitable to control the release of the active ingredient onto the active ingredient containing layer of the component prepared in accordance with step (h),
(k) performing steps (e) to (g),
(l) laminating the adhesive layer of the component prepared in step (k) onto the membrane layer of the component prepared in step (j), or *vice versa.*

In general, the expressions "active ingredient containing layer composition", "adhesive layer composition", and "membrane layer composition" mean that they comprise the same ingredients as described above for the "active ingredient containing layer", the "adhesive layer", and the "membrane layer". In addition thereto, they may comprise a suitable organic solvent, such as e.g. heptane or ethyl acetate, but other organic solvent or solvent mixtures known to the skilled person are also possible. Preferably, a volatile organic solvent or solvent mixture is used in the preparation of the respective compositions.

The preparation of the TDS of the present invention may be performed by firstly dispersing or dissolving, respectively (unless the polymer is already dissolved) the components for the active ingredient containing layer, that is the active ingredient and the matrix-forming polymer or copolymer, respectively, or a mixture thereof, in a suitable organic solvent as described above, such as e.g. heptane or ethyl acetate, or other organic solvent or solvent mixtures known to the skilled person. Preferably, a volatile organic solvent or solvent mixture is used. Optionally, further excipients as described in connection with the active ingredient containing layer are added. Typically, the matrix-forming polymer or copolymer or the mixture thereof is already present in a solvent. Here, according to the invention a polymer and/or copolymer is used that is as defined above in connection with the TDS according to the invention. The embodiments of the polymer matrix mentioned above as being preferred correspondingly apply to the method according to the invention.

Then, said mixture is applied onto a release liner (i) as a uniform layer and dried.

In the next step, a backing layer is applied to the active ingredient containing layer.

In a separate step, the adhesive layer is prepared by dispersing the polymer mixture forming the contact adhesive (and dissolved in an organic solvent), optionally together with further excipients as described in connection with the adhesive layer, in a suitable organic solvent such as e.g. heptane or ethyl acetate, but also other organic solvent or solvent mixtures known to the skilled person are possible. Preferably, a volatile organic solvent or solvent mixture is used. Then, said mixture is applied onto a release liner (ii) and allowed to dry. The embodiments of the adhesive layer mentioned above as being preferred correspondingly apply to the method according to the invention.

The components obtained in these two process steps are subsequently laminated together, namely such that the adhesive layer is directly applied to the active ingredient containing layer after the release liner (i) has been removed. In the embodiments where a membrane layer controlling the release of the active ingredient is used, the adhesive layer is laminated to the membrane, or *vice versa.*

In case that a membrane controlling the release of the active substance is to be applied, after the active ingredient containing layer is dried this can be applied to the side of the active ingredient containing layer after the release liner (i) has been removed. The adhesive layer is then laminated onto the membrane layer, or *vice versa.* Subsequently, pieces of the desired size can be punched from the finished laminate and packaged.

In the individual process steps, the organic solvents required to dissolve or disperse the respective components are removed by subjecting the products to a drying step by use of increasing temperatures, optionally also using a partial vacuum.

The preparation of the transdermal therapeutic systems in accordance with the present invention is further illustrated in the examples.

### Further aspects and embodiments

Preferably, no permeation enhancer is used in the TDS of the present invention. It is particularly preferred that the TDS of the present invention does not contain the permeation enhancers used in the transdermal therapeutic system disclosed in WO'241.

The size of the TDS of the present invention is not particularly limited, but is typically in the range from 4 to 40 cm². In an embodiment, the size of the TDS of the present invention is not larger than 36 cm², preferably not larger than 25 cm², more preferably not larger than 22 cm², most preferably not larger than 20 cm², such as e.g. 18, 16, 14, 12, 10, 8, 6 cm².

The daily amount of the active ingredient lumateperone delivered transdermally *in vivo* is preferably at least about 1.5 mg/day, preferably about 1.8 mg/day. This corresponds to a daily oral dose of 60 mg lumateperone tosylate being equivalent to 42 mg lumateperone free base, which is required taking into account the low oral bioavailability of about 4.4% of Caplyta^{®}.

In accordance therewith, the flux is preferably at least 3.75 µg/cm²*h. For a patch having a preferred size of 20 cm², this results in a daily amount of at least 1.5 mg/day. This flux is preferably achieved without the use of permeation enhancers in the TDS.

The application period of a TDS according to the invention is at least one day, preferably at least 2, more preferably at least 3 days. The TDS according to the first aspect of the present invention is preferably suitable for an application period of 2 to 3 days, more preferably 3 to 4 days. The TDS according to the second aspect of the present invention is preferably suitable for an application period of 2 to 3 days, more preferably 3 to 4 days. The TDS according to the third aspect of the present invention is preferably suitable for an application period of 2 to 3 days, more preferably 3 to 4 days.

Preferably, the TDS according to the invention has a constant permeation profile for several days. Here, the permeation is constant over a period of at least 24 hours (1 day), preferably at least 48 hours (2 days), more preferably at least 72 hours (3 days).

The TDS according to the first aspect of the present invention preferably has a constant permeation profile over a period of preferably 2 days, more preferably 3 days.

In a further aspect, the present invention concerns the use of (1) a polyisobutene, (2) a styrene-butadiene-styrene copolymer, (3) a silicone-acrylate hybrid polymer, (4) a styrene-isoprene-styrene copolymer, and mixtures thereof for stabilizing lumateperone free base in a transdermal therapeutic system, or reducing, preferably preventing degradation of lumateperone free base in a transdermal therapeutic system, preferably over a time period of at least 6 months, more preferably over a time period of at least 12 months at room temperature. The transdermal therapeutic system is preferably the TDS according to the present invention in terms of all of its aspects and embodiments.

In a further aspect of the present invention, the transdermal therapeutic system of the present invention is for use in a method for treating a disease selected from the group consisting of major depressive disorder (MDD) and adjunctive mixed features associated therewith, bipolar depression I or II and schizophrenia.

In a further aspect of the present invention, lumateperone is for use in a method for treating a disease selected from the group consisting of major depressive disorder (MDD) and adjunctive mixed features associated therewith, bipolar depression I or II and schizophrenia, wherein lumateperone is administered in free base form or salt form through the skin by means of the transdermal therapeutic system of the present invention.

### Specific embodiments

In a specific embodiment, the TDS of the present invention comprises (in accordance with the first aspect of the invention):
a. a backing layer, which is preferably a polyester foil;
b. an active ingredient containing layer, comprising a polymer matrix containing 10 to 15 wt.-%, preferably 15 wt.-%, of the active ingredient lumateperone, preferably in its free base form, and a mixture of two polyisobutenes (such as e.g. the product Duro-Tak^{®} 87-626A and 87-6900); the active ingredient containing layer preferably contains 0.05-2 wt.-% of the antioxidant tocopherol, ascorbyl palmitate or a combination thereof in order to increase the chemical stability of the active ingredient; the active ingredient containing layer optionally contains polybutene in an amount of 15-45 wt.-%; and the active ingredient containing layer optionally contains a crystallization inhibitor (such as PVP VA 64); for obtaining a 3 day patch, the matrix weight is preferably 65-80 g/m²;
c. a release liner, which is preferably a siliconized polyester foil.

In a further specific embodiment, the TDS of the present invention comprises (in accordance with the first aspect of the invention):
a. a backing layer, which is preferably a polyester foil;
b. an active ingredient containing layer, comprising a polymer matrix containing 10 to 15 wt.-%, preferably 15 wt.-%, of the active ingredient lumateperone, preferably in its free base form, and a styrene-butadiene-styrene polymer, preferably comprising a petroleum-based hydrocarbon resin (such as e.g. in the product Duro-Tak^{®} 87-6911); the active ingredient containing layer may contain a crystallization inhibitor (such as PVP); the active ingredient containing layer can contain a permeation enhancer (such as oleyl oleate) in order to increase permeation of the system - however, it is preferred that no permeation enhancer is added; for obtaining a 3-4 days patch, the matrix weight is preferably 65-80 g/m²;
c. a release liner, which is preferably a fluorosiliconized or a non-fluoro containing polyester foil.

In a further specific embodiment, the TDS of the present invention comprises (in accordance with the first aspect of the invention):
a. a backing layer, which is preferably a polyester foil;
b. an active ingredient containing layer, comprising a polymer matrix containing 10 to 15 wt.-%, preferably 15 wt.-%, of the active ingredient lumateperone, preferably in its free base form, and a silicone-acrylate hybrid polymer (e.g. BIO-PSA 7-6302) or a mixture of a silicone-acrylate hybrid polymer with low high tack (such as e.g. BIO-PSA 7-6302) and a silicone-acrylate hybrid polymer with high tack (such as e.g. BIO-PSA 7-6102), preferably in a wt.-ratio of 60-80:40-20; the active ingredient containing layer preferably contains 0.05-2 wt.-% of the antioxidant tocopherol, ascorbyl palmitate, or a combination thereof in order to increase the chemical stability of the active ingredient; for obtaining a 3 day patch, the matrix weight is preferably 65-80 g/m²;
c. a release liner, which is preferably a fluoropolymer-coated or fluorosiliconized, or a non-perfluoro or non-polyfluoro polyester foil.

In a further specific embodiment, the TDS of the present invention comprises (in accordance with the first aspect of the invention):
a. a backing layer, which is preferably a polyester foil;
b. an active ingredient containing layer, comprising a polymer matrix containing 10 to 15 wt.-%, preferably 15 wt.-%, of the active ingredient lumateperone, preferably in its free base form, and a styrene-isoprene-styrene polymer and a alicyclic hydrogenated hydrocarbon resin, preferably in a wt.-ratio of about 1:1; the active ingredient containing layer preferably contains 0.05-2 wt.-% of the antioxidant tocopherol, ascorbyl palmitate, or a combination thereof in order to increase the chemical stability of the active ingredient; for obtaining 3 day patch, the matrix weight is preferably 70-80 g/m²;
c. a release liner, which is preferably a fluoropolymer-coated or fluorosiliconized, or a non-perfluoro or non-polyfluoro polyester foil.

In a further specific embodiment, the TDS of the present invention comprises (in accordance with the second aspect of the invention):
a. a backing layer as defined above
b. an active ingredient containing layer as defined above
c. an adhesive layer, comprising a polyacrylate adhesive with free hydroxy groups (such as e.g. Duro-Tak^{®} 387-2516), preferably with a small amount of e.g. 2 wt.-% a of a mixture of decanoyl and octanoyl glycerides (such as e.g. Miglyol^{®} 812) added in order to increase the adhesiveness of the layer; alternatively comprising a styrene-butadiene-styrene (SBS) copolymer adhesive, preferably further comprising a petroleum-based hydrocarbon resin (such as e.g. in the product Duro-Tak^{®} 87-6911); or alternatively containing a mixture of one or more polyisobutylene (such as e.g. the products Duro-Tak^{®} 87-626A and Duro-Tak^{®} 87-6900 in a ratio of 1:1 or 3:2) and one or more polybutene (such as e.g. the products Indopol H-1900 and Indopol H-18000 in a ratio of 1:1 or 3:2), wherein the polyisobutylene/polybutene ratio is 1:1); for obtaining a 3 to 4 days patch, the weight of the adhesive layer is preferably about 30 g/m²;
d. a release liner as defined above.

In a further specific embodiment, the TDS of the present invention comprises (in accordance with the third aspect of the invention):
a. a backing layer as defined above;
b. an active ingredient containing layer as defined above;
c. a membrane layer consisting of coherent polyethylene (such as e.g. the product Cotran^{™} 9719), or alternatively, consisting of porous polypropylene (such as e.g. the product Celgard^{™} 2400).
d. an adhesive layer as defined above
e. a release liner as defined above.

### Examples

### Example 1: Monolayer formulation (first aspect of the invention)

### a) Preparation

### Example 1.1: Polyisobutene polymer matrix

### Samples 1-7

An active ingredient containing layer containing the active ingredient lumateperone free base, a polyisobutene (Duro-Tak^{®} 87-626A, Duro-Tak^{®} 87-625A, Duro-Tak^{®} 87-6900, and mixtures thereof), and, optionally, further excipients (Indopol H-1900, Indopol H-18000, PVP, oleyl oleate), was coated on a protective film (Primeliner^{™} 78HL). The solvents were removed in a drying oven. The active layer was then laminated with a backing layer (Hostaphan^{™} MN 19 Med or Scotchpak^{™} 9738). The resulting laminates are shown in **Table 1.**

### Example 1.2: Styrene-butadiene-styrene (SBS) copolymer matrix

### Samples 8-26

An active ingredient containing layer containing the active ingredient lumateperone free base, a styrene-butadiene-styrene copolymer adhesive including resin (Duro-Tak^{®} 87-6911 or Duro-Tak^{®} 87-6173), and, optionally, further excipients (PVP K90, oleyl oleate, isopropylmyristate, lauyryl lactate, 1-dodecanol, oleic acid, DEET, Trancutol^{®}, Miglyol^{®} 812, Span^{®} 60), was coated on a protective film (Primeliner^{™} 78HL). The solvents were removed in a drying oven. The active layer was then laminated with a backing layer (Hostaphan^{™} MN 19 Med or Scotchpak^{™} 9738). The resulting laminates are shown in **Table 1.**

### Example 1.3: Silicone-acrylate hybrid polymer matrix

### Samples 27-30

An active ingredient containing layer containing the active ingredient lumateperone free base, a silicone-acrylate hybrid polymer (BIO-PSA 7-6302, BIO-PSA 7-6102, BIO-PSA 7-6301, or BIO-PSA 7-6101) was coated on a protective film (Scotchpak^{™} 9709). The solvents were removed in a drying oven. The active layer was then laminated with a backing layer (Scotchpak^{™} 9738). The resulting laminates are shown in **Table 1.**

### Example 1.4: Styrene-isoprene-styrene (SIS) copolymer matrix

### Samples 31-33

An active ingredient containing layer containing the active ingredient lumateperone free base, a styrene-isoprene-styrene copolymer, alicyclic hydrogenated hydrocarbon resin (Arkon^{®} P-100), and, optionally, further excipients (tocopherol, or paraffin)), was coated on a protective film (Primeliner^{™} 78HL). The solvents were removed in a drying oven. The active layer was then laminated with a backing layer (Scotchpak^{™} 9738). The resulting laminates are shown in the following **Table 1.**

**Table 1**

| **No.** | **Batch** | **Composition of the (dried) active ingredient containing (matrix) layer** | **Matrix weight [g/m²]** | **Backing layer/ Release liner** |
|---|---|---|---|---|
| 1 | 005LUMTDS | 10% Lumateperone, 67.5% Duro-Tak^{®} 87-6626A, 22.5% Duro-Tak^{®} 87-625A | 50 | Hostaphan MN12/ Primeliner 78HL |
| 2 | 018LUMTDS | 15% Lumateperone, 42.5% Duro-Tak^{®} 87-6626A, 42.5% Duro-Tak^{®} 87-6900 | 50 | Scotchpak 9738/ Primeliner 78HL |
| 3 | 019LUMTDS | 15% Lumateperone 22.5% Duro-Tak^{®} 87-6626A, 22.5% Duro-Tak^{®} 87-6900, 40% Indopol H-1900 | 50 | Scotchpak 9738/ Primeliner 78HL |
| 4 | 020LUMTDS | 15% Lumateperone 22.5% Duro-Tak^{®} 87-6626A, 22.5% Duro-Tak^{®} 87-6900, 40% Indopol H-18000 | 50 | Scotchpak 9738/ Primeliner 78HL |
| 5 | 021LUMTDS | 15% Lumateperone 22.5% Duro-Tak^{®} 87-6626A, 22.5% Duro-Tak^{®} 87-6900, 20% Indopol H-1900, 20% Indopol H-18000 | 50 | Scotchpak 9738/ Primeliner 78HL |
| 6 | 026LUMTDS | 15% Lumateperone 38.75% Duro-Tak^{®} 87-6626A, 38.75% Duro-Tak^{®} 87-6900, 7.5% PVP K90 | 50 | Scotchpak 9738/ Primeliner 78HL |
| 7 | 027LUMTDS | 15% Lumateperone 38.75% Duro-Tak^{®} 87-6626A, 38.75% Duro-Tak^{®} 87-6900, 7.5% oleyl oleate | 50 | Scotchpak 9738/ Primeliner 78HL |
| | | | | |
| 8 | 006LUMTDS | 10% Lumateperone, 90% Duro-Tak^{®} 87-6911 | 50 | Hostaphan MN12/ Primeliner 78HL |
| 9 | 078LUMTDS | 10% Lumateperone, 90% Duro-Tak^{®} 87-6911 | 50 | Scotchpak 9738/ Primeliner 78HL |
| | | | 80 | |
| | | | 100 | |
| 10 | 083LUMTDS | 10% Lumateperone, 90% Duro-Tak^{®} 87-6911 | 125 | Scotchpak 9738/ Primeliner 78HL |
| 11 | 011LUMTDS | 15% Lumateperone, 85% Duro-Tak^{®} 87-6911 | 50 | Hostaphan MN12/ Primeliner 78HL |
| 12 | 016LUMTDS | 15% Lumateperone, 85% Duro-Tak^{®} 87-6911 | 50 | Scotchpak 9738/ Primeliner 78HL |
| 13 | 077LUMTDS | 15% Lumateperone, 85% Duro-Tak^{®} 87-6911 | 65 | Hostaphan MN12/ Primeliner 78HL |
| | | | 80 | |
| 14 | 025LUMTDS | 15% Lumateperone, 85% Duro-Tak^{®} 87-6911 | 100 | Scotchpak 9738/ Primeliner 78HL |
| | | | 125 | |
| 15 | 031LUMTDS | 15% Lumateperone, 77.5% Duro-Tak^{®} 87-6911, 7.5% PVP | 50 | Scotchpak 9738/ Primeliner 78HL |
| 16 | 064LUMTDS | 10% Lumateperone, 90% Duro-Tak^{®} 87-6173 | 50 | Scotchpak 9738/ Primeliner 78HL |
| | | | 65 | |
| | | | 80 | |
| 17 | 032LUMTDS | 15% Lumateperone, 85% Duro-Tak^{®} 87-6173 | 50 | Scotchpak 9738/ Primeliner 78HL |
| | 063LUMTDS | | 65 | |
| | 063LUMTDS | | 80 | |
| | 104LUMTDS | | 80 | |
| 18 | 106LUMTDS | 15% Lumateperone, 80% Duro-Tak^{®} 87-6911, 5% oleyl oleate | 80 | Scotchpak 9738/ Primeliner 78HL |
| 19 | 107LUMTDS | 15% Lumateperone, 80% Duro-Tak^{®} 87-6911, 5% IPM (isopropyl myristate) | 80 | Scotchpak 9738/ Primeliner 78HL |
| 20 | 108LUMTDS | 15% Lumateperone, 80% Duro-Tak^{®} 87-6911, 5% lauryl lactate | 80 | Scotchpak 9738/ Primeliner 78HL |
| 21 | 109LUMTDS | 15% Lumateperone, 80% Duro-Tak^{®} 87-6911, 5% 1-dodecanol | 80 | Scotchpak 9738/ Primeliner 78HL |
| 22 | 110LUMTDS | 15% Lumateperone, 80% Duro-Tak^{®} 87-6911, 5% oleic acid | 80 | Scotchpak 9738/ Primeliner 78HL |
| 23 | 111LUMTDS | 15% Lumateperone, 80% Duro-Tak^{®} 87-6911, 5% DEET (diethyl-m-toluamide) | 80 | Scotchpak 9738/ Primeliner 78HL |
| 24 | 112LUMTDS | 15% Lumateperone, 80% Duro-Tak^{®} 87-6911, 5% Trancutol^{®} | 80 | Scotchpak 9738/ Primeliner 78HL |
| 25 | 113LUMTDS | 15% Lumateperone, 80% Duro-Tak^{®} 87-6911, 5% Miglyol^{®} 812 | 80 | Scotchpak 9738/ Primeliner 78HL |
| 26 | 114LUMTDS | 15% Lumateperone, 80% Duro-Tak^{®} 87-6911, 5% Span^{®} 60 | 80 | Scotchpak 9738/ Primeliner 78HL |
| | | | | |
| 27 | 017LUMTDS | 15% Lumateperone, 85% BIO-PSA 7-6302 | 50 | Scotchpak 9738/ Scotchpak 9709 |
| 28 | 028LUMTDS | 15% Lumateperone, 85% BIO-PSA 7-6102 | 50 | Scotchpak 9738/ Scotchpak 9709 |
| 29 | 029LUMTDS | 15% Lumateperone, 85% BIO-PSA 7-6301 | 50 | Scotchpak 9738/ Scotchpak 9709 |
| 30 | 030LUMTDS | 15% Lumateperone, 85% BIO-PSA 7-6101 | 50 | Scotchpak 9738/ Scotchpak 9709 |
| | | | | |
| 31 | 115LUMTDS | 15% Lumateperonem 0.15% tocopherol, 25.46% SIS blockcoplymer, 59.39% Arkon^{®} P-100 | 70 | Scotchpak 9738/ Primeliner 78HL |
| 32 | 116LUMTDS | 15% Lumateperonem 0.05% tocopherol, 42.48% SIS blockcoplymer, 42.48% Arkon^{®} P-100 | 60 | Scotchpak 9738/ Primeliner 78HL |
| 33 | 117LUMTDS | 15% Lumateperonem 7.5% paraffin oil, 23.25% SIS blockcoplymer, 54.25% Arkon^{®} P-100 | 80 | Scotchpak 9738/ Primeliner 78HL |

### b) Crystallization and phase system

Some of the samples prepared in Example 1.1-1.4 were analyzed for the occurrence of crystallization and the presence of a biphasic system after being stored at 40°C/75% RH. The results are shown in the following **Table 2.**

**Table 2**

| **No.** | **Batch** | **Storage conditions** | **Crystallization visible?** | **Biphasic system visible (using a microscope)?** |
|---|---|---|---|---|
| 1 | 005LUMTDS | 12 months at 40°C/75% RH | yes | no |
| 2 | 018LUMTDS | 6 months at 40°C/75% RH | no | no |
| 3 | 020LUMTDS | 6 months at 40°C/75% RH | no | no |
| 4 | 021LUMTDS | 6 months at 40°C/75% RH | no | no |
| 5 | 011LUMTDS | 12 months at 40°C/75% RH | no | no |
| 6 | 016LUMTDS | 6 months at 40°C/75% RH | no | no |
| 7 | 031LUMTDS | 6 months at 40°C/75% RH | no | yes |
| 8 | 032LUMTDS | 6 months at 40°C/75% RH | no | no |
| 9 | 017LUMTDS | 6 months at 40°C/75% RH | no | no |
| 10 | 028LUMTDS | 6 months at 40°C/75% RH | no | no |
| 11 | 029LUMTDS | 6 months at 40°C/75% RH | no | no |
| 12 | 030LUMTDS | 6 months at 40°C/75% RH | no | no |
| 13 | 117LUMTDS | 1 month at 40°C/75% RH | no | no |

| | | | | |
|---|---|---|---|---|
| rt = room temperature; RH = relative humidity | | | | |

### c) Adhesive strength

The adhesive strength was measured after 0 months, 1 months, 3 months and 6 months at 40°C/75% RH for some of the transdermal therapeutic systems prepared in Examples 1.1-1.4.

The adhesion strength can be determined as the force required to detach a specimen from a suitable support at a specified angle and at a defined speed. For the determination of the adhesion strength, TDS of a defined size, e.g. 10 cm², are punched out and conditioned at 23 ± 1°C and 50 ± 5% RH. A conductive strip, e.g. 10 cm², is applied to the TDS. The conductive strip consists of, e.g. double-sided adhesive tape. The TDS is applied to a test plate, e.g. made of steel, with its release liner removed and then pressed between two glass plates for e.g. 1 min with a 2 kg weight. The test plate is fixed horizontally in a tensile testing machine, e.g. Texture Analyzer plus from Stable Micro Systems, and the liner is clamped so that the TDS is pulled off at a 90° angle. The measurement is made at a specified speed of usually 300 ± 30 mm/min at typically 23 ± 1°C and 50 ± 5% RH. The mean force [N/25 mm], normalized to a specimen width of 25 mm, measured over the distance is the adhesive force.

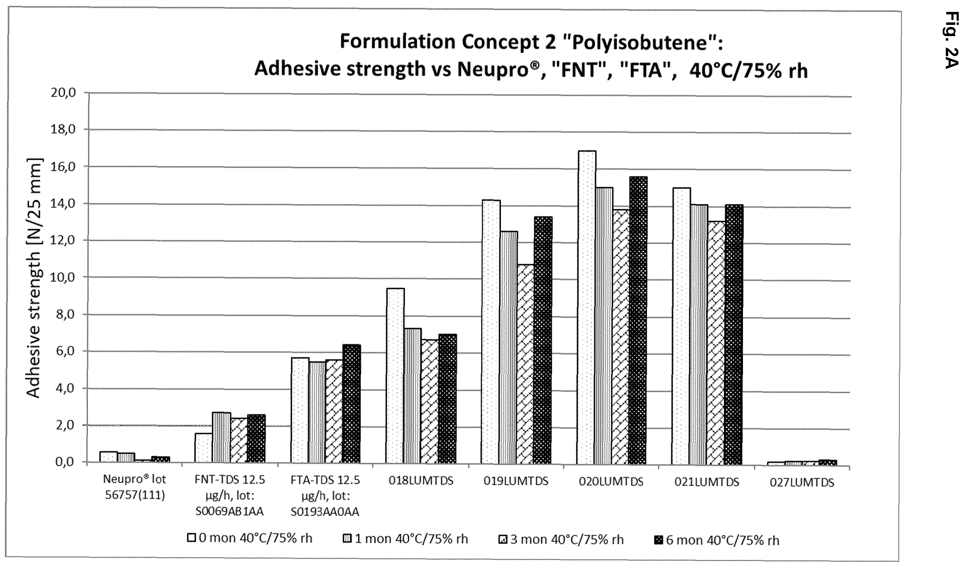

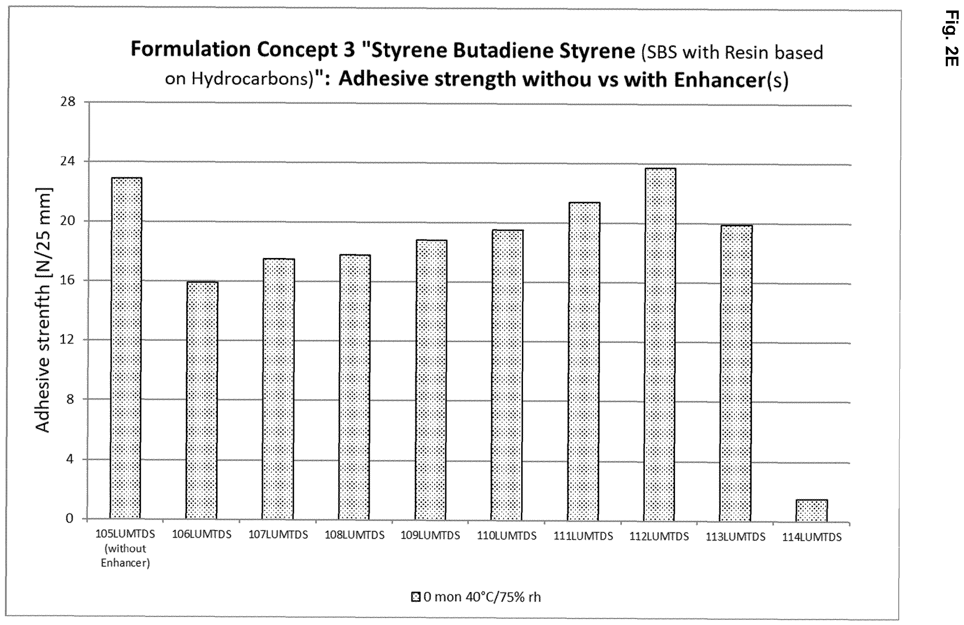

The results are shown in Fig. 2A-D.

From Fig. 2A-D, it can be taken that the TDS comprising lumateperone free base in combination with (1) a polyisobutene polymer (Duro-Tak^{®} 87-625A, Duro-Tak^{®} 87-626A, Duro-Tak^{®} 87-690, and mixtures thereof), (2) a styrene-butadiene-styrene copolymer (Duro-Tak^{®} 87-6911), (3) a silicone-acrylate hybrid polymer (BIO-PSA 7-6302, or BIO-PSA 7-6102) or (4) a styrene-isoprene-styrene copolymer has a higher adhesive strength than the 1-day patch Neupro^{®} and the 3-day patches FNT and FTA.

In particular, it can be seen from **Fig 2A** that a significantly higher adhesive strength can be achieved with polybutene (both with Indopol H-1900 and H-18000, either alone or in combination). All measured formulations resulted in storage-stable formulations with regard to adhesive strength. The use of oleyl oleate in a concentration of 7.5 % surprisingly shows a dramatic reduction in adhesive strength. Thus, the use of oleyl oleate is not preferred.

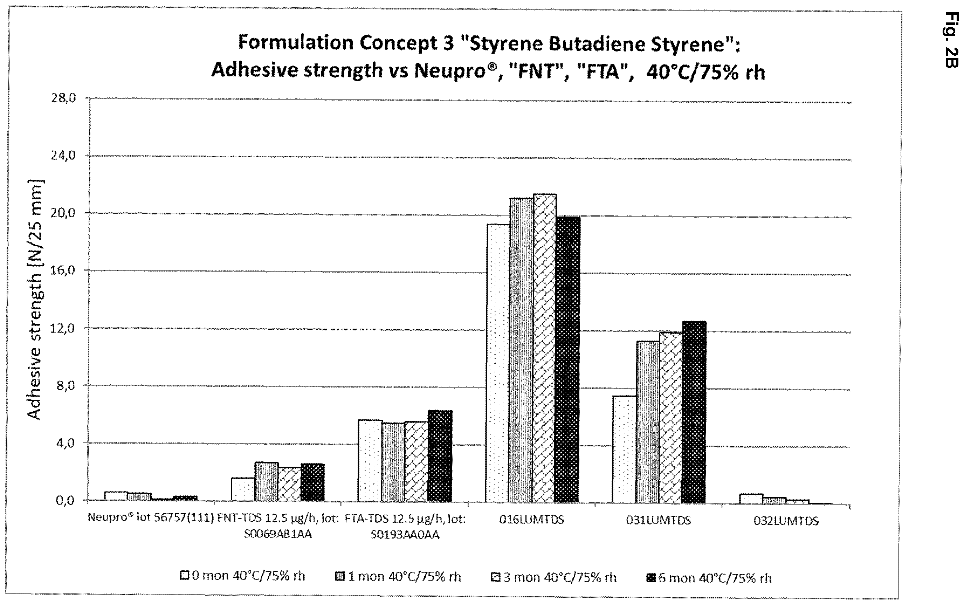

From **Fig. 2B****,** it can be seen that a significantly higher adhesive strength can be achieved for a TDS comprising a SBS with petroleum-based hydrocarbon resin (Duro-Tak^{®} 87-6911) than the 1-day patch Neupro^{®} and the 3-day patches FNT and FTA. The use of hydrated glycerin ester resins (Duro-Tak^{®} 87-6173) reduces the adhesive force of the TDS. Furthermore, the use of SPAN^{®} 60 reduces the adhesive force.

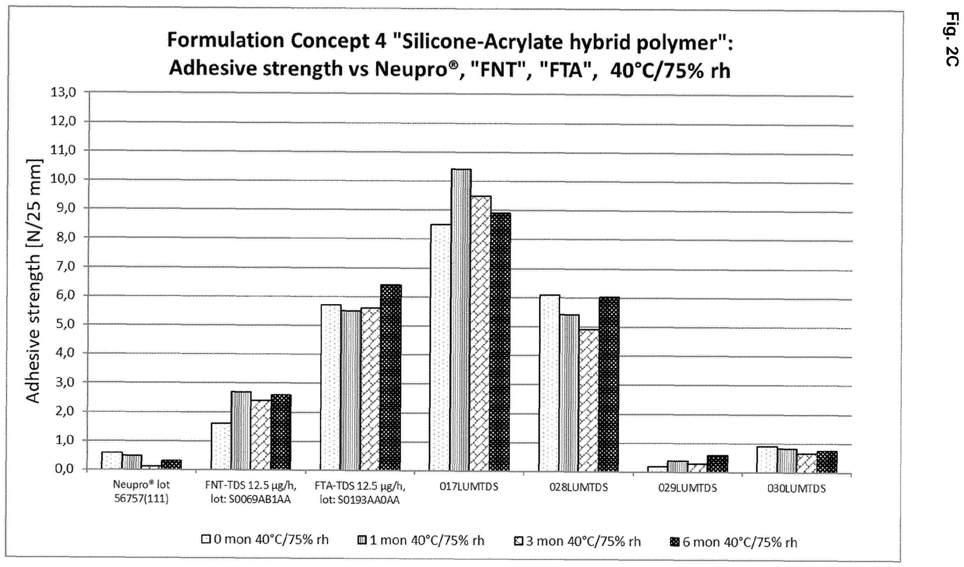

From **Fig. 2C****,** it can be seen that in particular the high tack silicone-acrylate hybrid polymer in ethyl acetate (BIO-PSA 7-6302) resulted in significantly higher adhesive strengths than the 1-day patch Neupro^{®} and the 3-day patches FNT and FTA. The use of silicone-acrylate hybrid polymer in hexane (BIO-PSA 7-6301 and BIO-PSA 7-6101) did not result in significantly higher adhesive strengths than the 1-day patch Neupro^{®} and the 3-day patches FNT and FTA.

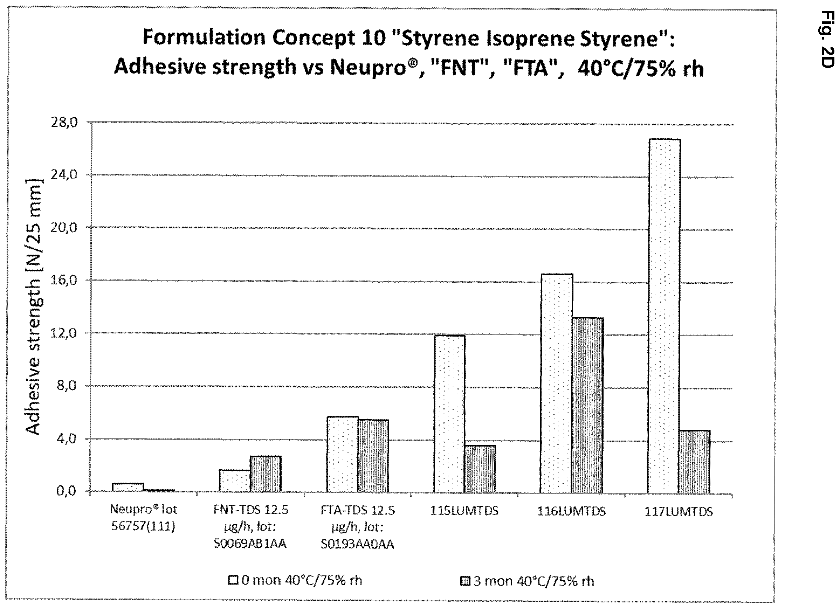

From **Fig. 2D****,** it can be seen that the use of all styrene-isoprene-styrene copolymers resulted in significantly higher adhesive strengths than the 1-day patch Neupro^{®} and the 3-day patches FNT and FTA. Furthermore, the initial adhesive strength (at 0 months) could be increased by increasing the ratio of alicyclic hydrated hydrocarbon resin to SIS copolymer or by using viscous paraffin oil. However, the adhesive strength after storage (after 1 month) decreased for batch no. 33 containing a large amount of alicyclic hydrated hydrocarbon resin. Thus, an optimal ratio is at about 1:1 of alicyclic hydrated hydrocarbon resin to SIS copolymer.

There is no relevant decrease in adhesive strength during storage over 6 months at 40°C/75% RH.

### d) Tack

The tack was measured after 0 months, 1 months, 3 months and 6 months at 40°C/75% RH for some of the transdermal therapeutic systems prepared in Examples 1.1-1.4.

Tack can be determined as the maximum force required to completely separate a stainless steel specimen from the adhesive layer of a TDS. For the determination of Tack, a patch or laminate is conditioned at 23 ± 1°C and 50 ± 5% RH and subsequently fixed to a perforated carrier plate with its release liner removed and the adhesive matrix open. The plate is fixed in a tensile testing machine, e.g. Texture Analyser plus from Stable Micro Systems. At usually 23 ± 1°C and 50 ± 5% rH, the test specimen is pressed onto the top of the specimen and peeled off after a defined contact time of usually 2 sec. A series of measurements should be completed within 30 min after removal of the release liner from the first specimen. The maximum force (Tack; [N]) to separate the bond between the specimen and the adhesive layer is determined.

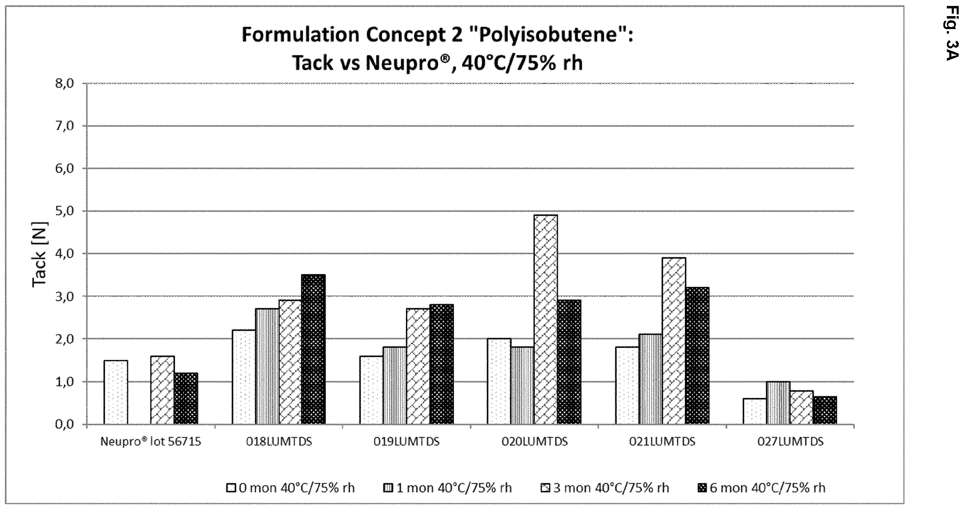

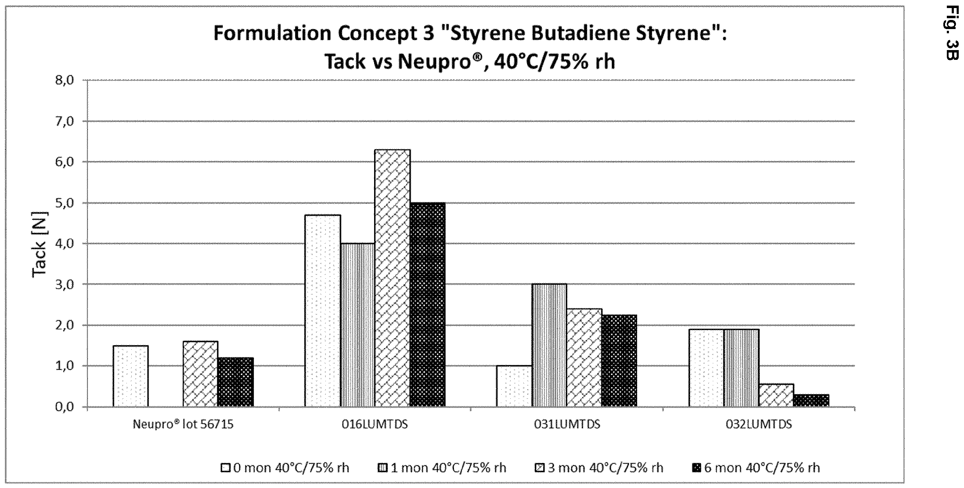

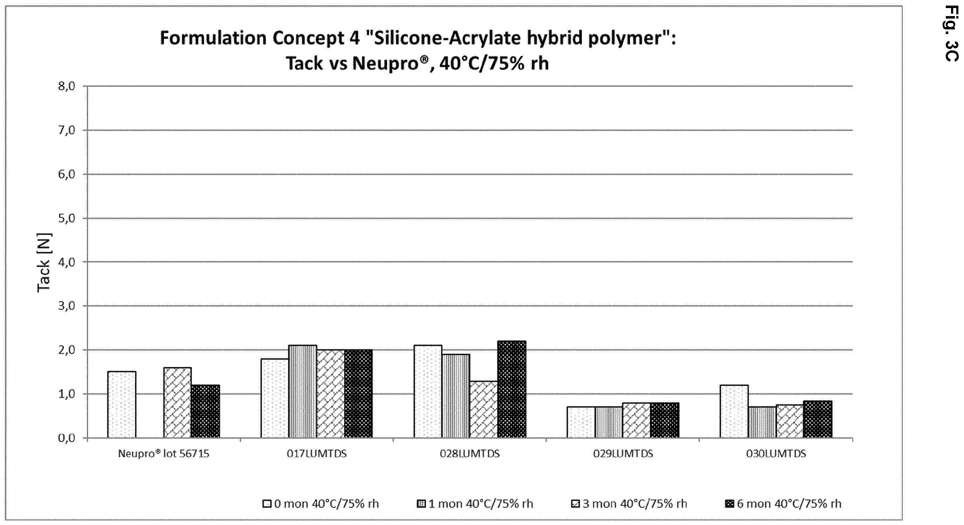

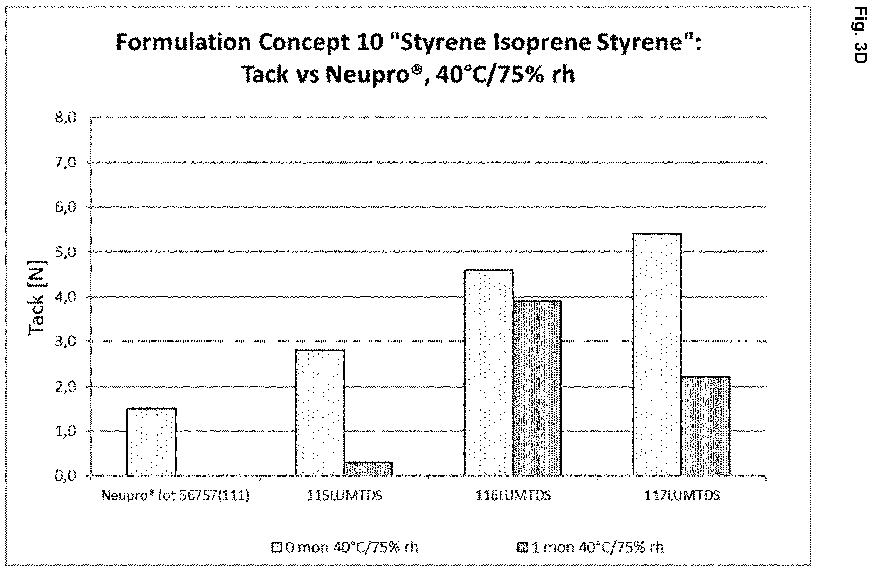

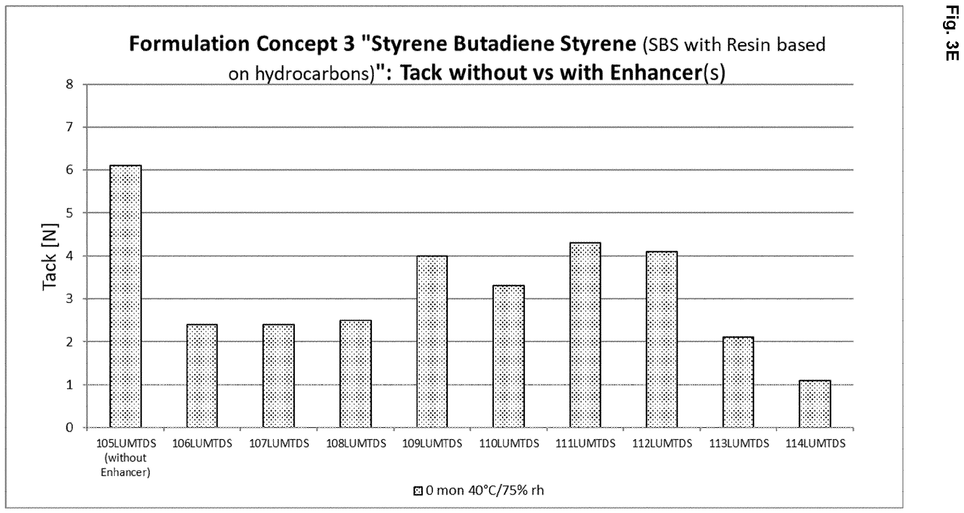

The results are shown in **Fig. 3A-3D****.**

From **Fig. 3A-3D****,** it can be taken that lumateperone free base in combination with (1) a polyisobutene polymer (Duro-Tak^{®} 87-625A, Duro-Tak^{®} 87-626A, Duro-Tak^{®} 87-690, and mixtures thereof), (2) a styrene-butadiene-styrene copolymer (Duro-Tak^{®} 87-6911), (3) a silicone-acrylate hybrid polymer (BIO-PSA 7-6302, or BIO-PSA 7-6102) or (4) a styrene-isoprene-styrene copolymer show a significantly higher tack than the 1-day patch Neupro.

There is no relevant decrease in tack during storage over 6 months at 40°C/75% RH.

### e) Separation force

The separation force, i.e. the force that is required to remove the release liner from the transdermal therapeutic system, was measured after 0 months, 1 months, 3 months and 6 months at 40°C/75% RH for some of the transdermal therapeutic systems prepared in Examples 1.1-1.4.

The separation force can be determined as the force required to detach a sample from its release liner at a specified angle and speed. For its determination, TDS's of a defined size, e.g. 10 cm², are punched out and conditioned at 23 ± 1°C and 50 ± 5% RH. A conductive strip, in the form of a liner, is cut out. A guide strip, the width of the TDS, is attached to the TDS. Then the TDS is fixed with the release liner facing downwards on an apparatus slide using double-sided adhesive tape. This is inserted into a tensile testing machine, e.g. Texture Analyser plus from Stable Micro Systems, in such a way that the TDS is pulled off at an angle of 90°. The speed is typically 300 ± 30 mm/min and is usually performed at 23 ± 1°C and 50 ± 5% RH. The average force [N/25 mm], normalized to a specimen width of 25 mm, measured across the separation distance is the separation force.

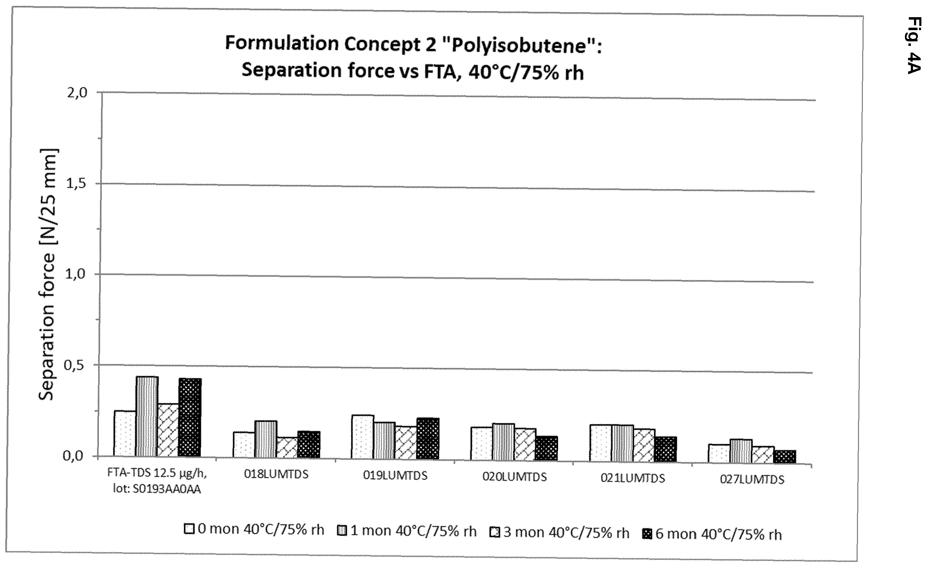

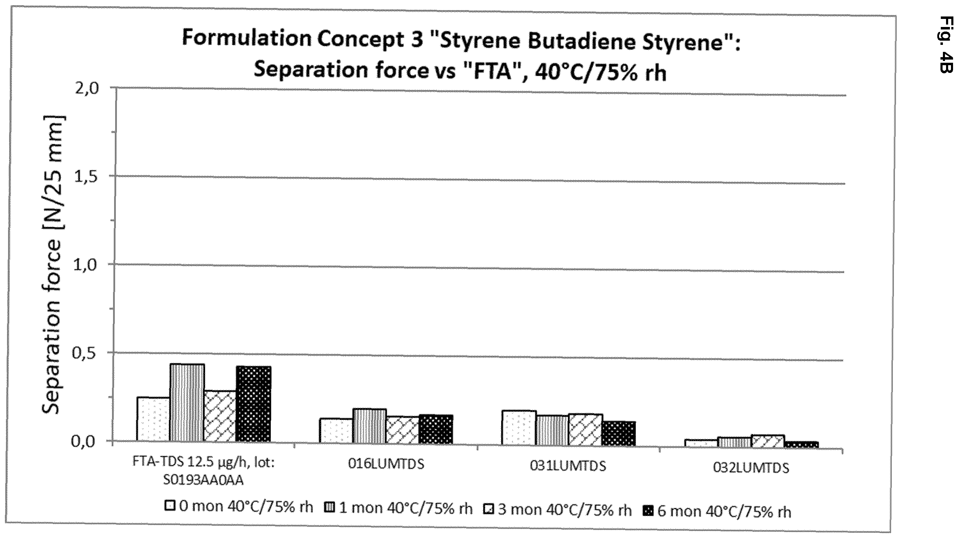

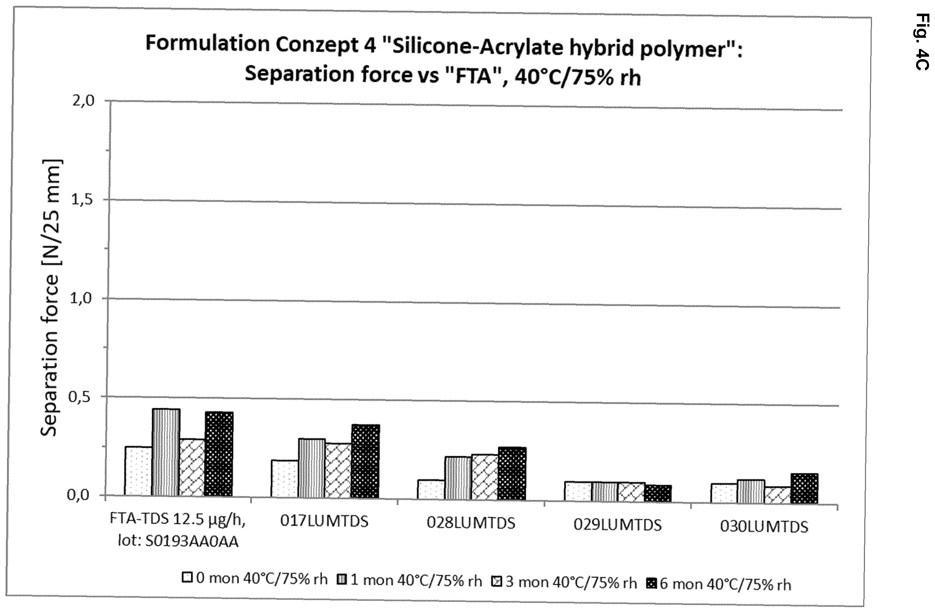

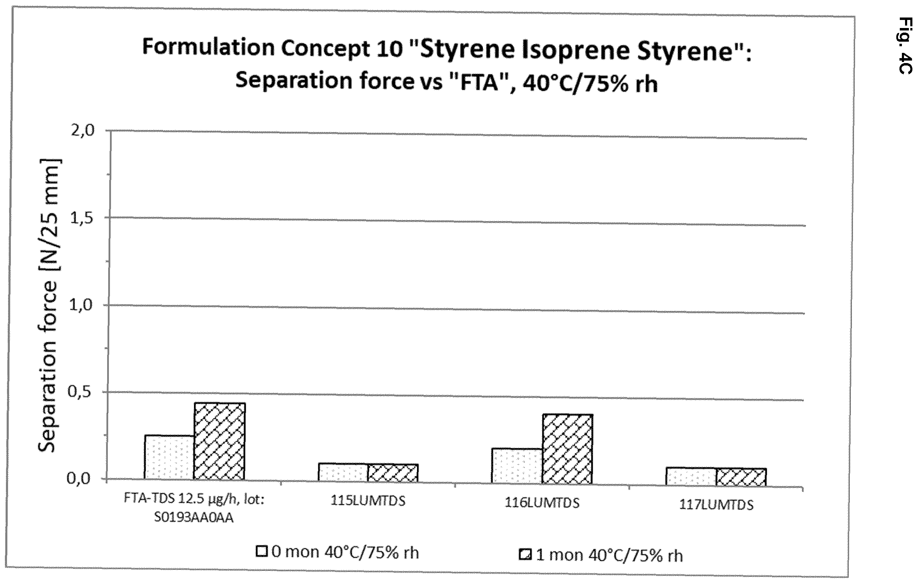

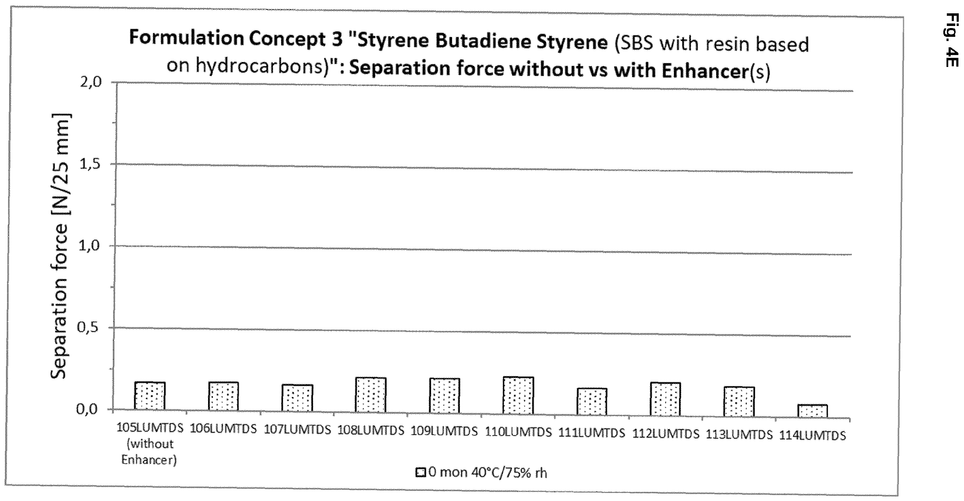

The results are shown in Fig. 4A-4D.

From Fig. 4A-4D, it can be taken that lumateperone free base in combination with (1) a polyisobutene polymer (Duro-Tak^{®} 87-625A, Duro-Tak^{®} 87-626A, Duro-Tak^{®} 87-690, and mixtures thereof), (2) a styrene-butadiene-styrene copolymer (Duro-Tak^{®} 87-6911 or Duro-Tak^{®} 87-6173 ), (3) a silicone-acrylate hybrid polymer (BIO-PSA 7-6302, BIO-PSA 7-6102, BIO-PSA 7-6301, or BIO-PSA 7-6101)) or (4) a styrene-isoprene-styrene copolymer leads to low separation forces, which moreover do not show any relevant increase in separation force during storage over 6 months at 40°C/75% RH by use of a siliconized polyester Release liner.

### f) Permeation

### aa)Preliminary remarks

In order to realize a small patch size, the cumulative permeated amount per 24 h must be sufficiently high. An amount of about 1.8 mg lumateperone must be provided transdermally at an oral bioavailability of about 4.4% per day.

In order to realize a small patch size of about 20 cm², a flux of at least 3.75 µg/cm²*h must be achieved (3.75 µg/cm²*h x 20 cm² x 24 = 1,800 µg/24 h = 1.8 mg/24 h).

For a unit area of 1 cm², theoretically 90 µg must be delivered transdermally per day, resulting in a cumulative permeated amount per cm² of 270 µg after 3 days, and 360 µg after 4 days.

Ideally, this flux is achieved without the use of permeation enhancers.

### bb)Permeation on human skin model (HSE)

The permeation of selected samples prepared in Examples 1.1-1.3 was measured on human skin model in order to determine the cumulatively permeated amount of lumateperone per cm² for a time period of up to 96 hours.

The *in vitro* skin permeation studies were performed using a skin permeation system from NovoCell Schönbach according to OECD (2004) Test Guideline 428 "Skin absorption: In vitro Method & Series on testing and assessment", No. 28 "Guidance document for the conduct of skin absorption studies".

A measuring cell was kept at 32 ± 1 °C throughout the measurement. The measuring cell consists of a donor and acceptor chamber, which are separated from each other by a heat-separated epidermis of human skin with an effective permeation area of 1.05 cm² lying on a cellulose membrane. The matrix of the patch to be tested (size about 1.2 cm²) was adhered to the stratum corneum, with the surface to be released facing the acceptor chamber. The measuring cell contained a total volume of 15 mL and was filled with phosphate buffer pH 4.5. At defined time points (e.g., 1, 2, 3, 6, 9, 12, 24, 36, 48, 72 and 96 hours), aliquots were taken as samples from the acceptor chamber, RP-HPLC analysis was used to determine the concentration of lumateperone, and immediately removed aliquot was replaced with fresh buffer. A homogeneous distribution of temperature and concentration of lumateperone was ensured by an integrated magnetic stirring system in the acceptor chamber.

RP-HPLC analysis and calculation of sample concentrations were performed. Stationary phase: C18 (e.g. 50x3 mm, 5 µm particle size, 30°C oven temperature). Mobile phase: 10mM phosphate buffer (pH 2.5)/methanol; 55/45 (v/v), with flow 0.7 mL/min. Injection volume: 10 µL, with detection at 229 nm, retention time lumateperone about 3-4 minutes, run time 5 minutes (isocratic). Evaluation was performed via 1-point calibration using external standard solution. Calculation of cumulative release [%] based on determined concentration in sample solutions.

Subsequently, the cumulative permeated amount per time point was calculated and plotted against time in a graph. Then the steady state flux can be calculated [µg/cm²/h].

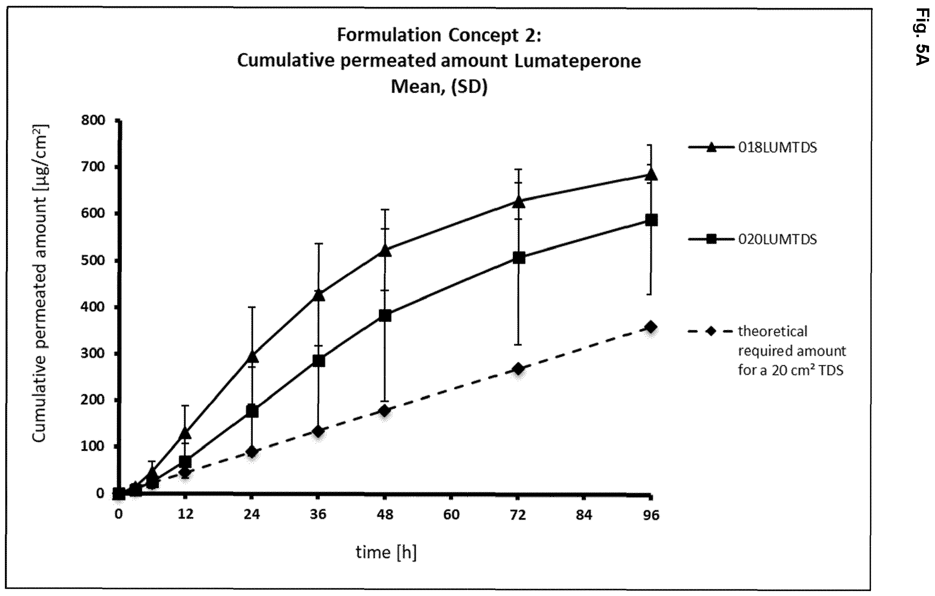

The results are depicted in **Fig. 5A-5D****.**

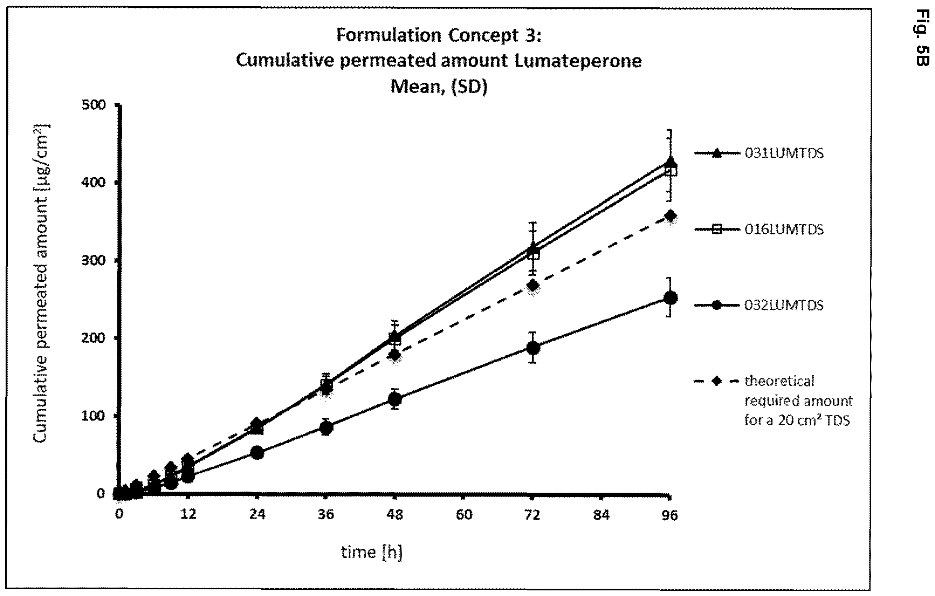

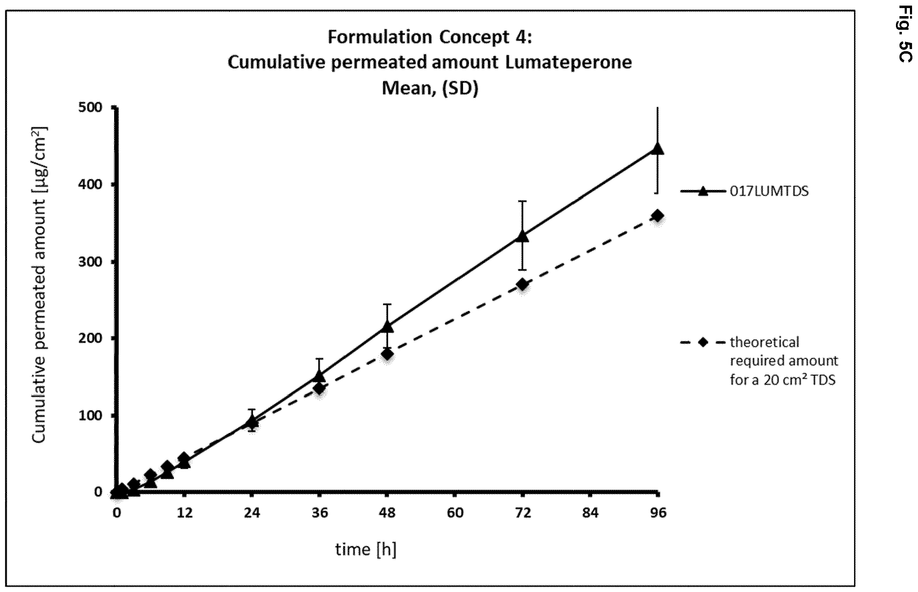

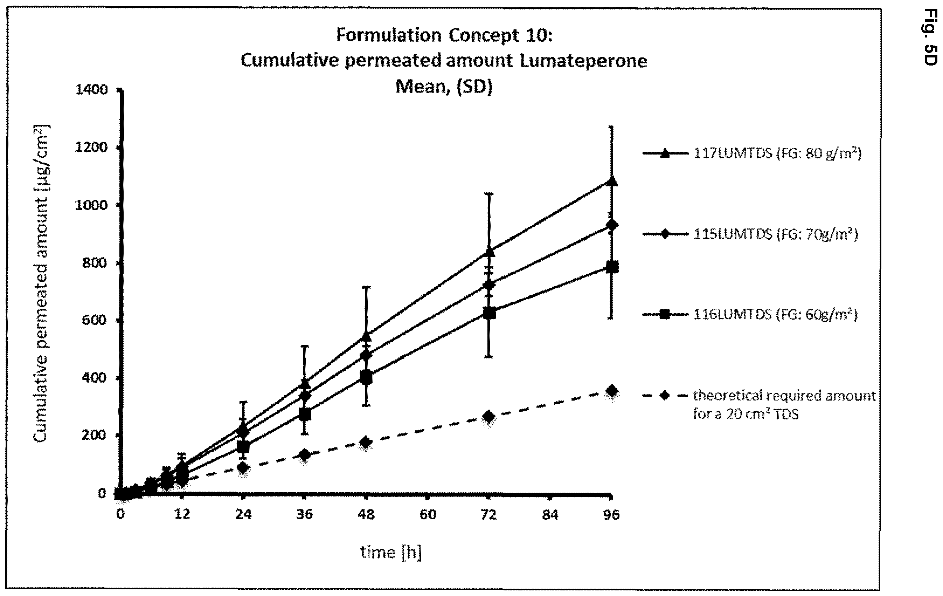

The samples show the following permeation effect on human skin model (HSE) from high to low in **Fig. 5****:**
For **Fig. 5A****:**
   15% Lumateperone, 42.5% Duro-Tak^{®} 87-6626A, 42.5% Duro-Tak^{®} 87-6900 (018LUMTDS) >
   15% Lumateperone, 22.5% Duro-Tak^{®} 87-6626A, 22.5% Duro-Tak^{®} 87-6900, 40% Indopol H-18000 (020LUMTDS)
For **Fig 5B****:**
   15% Lumateperone, 82.5% Duro-Tak^{®} 87-6911, 2.5% PVP (031LUMTDS) >
   15% Lumateperone, 85% Duro-Tak^{®} 87-6911 (016LUMTDS) >
   15% Lumateperone, 85% Duro-Tak^{®} 87-6173 (032LUMTDS).
For **Fig 5C****:**
   15% Lumateperone, 85% BIO-PSA 7-6302
For **Fig. 5D****:**
   15% Lumateperone, 7.5% paraffin oil, 23.25% SIS blockcoplymer, 54.25% Arkon^{®} P-100 >
   15% Lumateperonem 0.15% tocopherol, 25.46% SIS blockcoplymer, 59.39% Arkon^{®} P-100 >
   15% Lumateperonem 0.05% tocopherol, 42.48% SIS blockcoplymer, 42.48% Arkon^{®} P-100.

The dashed line in **Fig. 5A-5D** indicates the theoretically required amount of lumateperone for a 20 cm² matrix. Most of the samples comprising are in accordance with this requirement and only samples 032LUMTDS leads to a lower amount of permeated lumateperone (up to 96h).

It is not required to add an enhancer in order to fulfill the permeation requirement for a 20 cm² matrix.

The permeation for these patches (50 mg/cm² patches at 15% drug load) is constant for 2-3 days.

### cc) Optimization of drug concentration and matrix weight

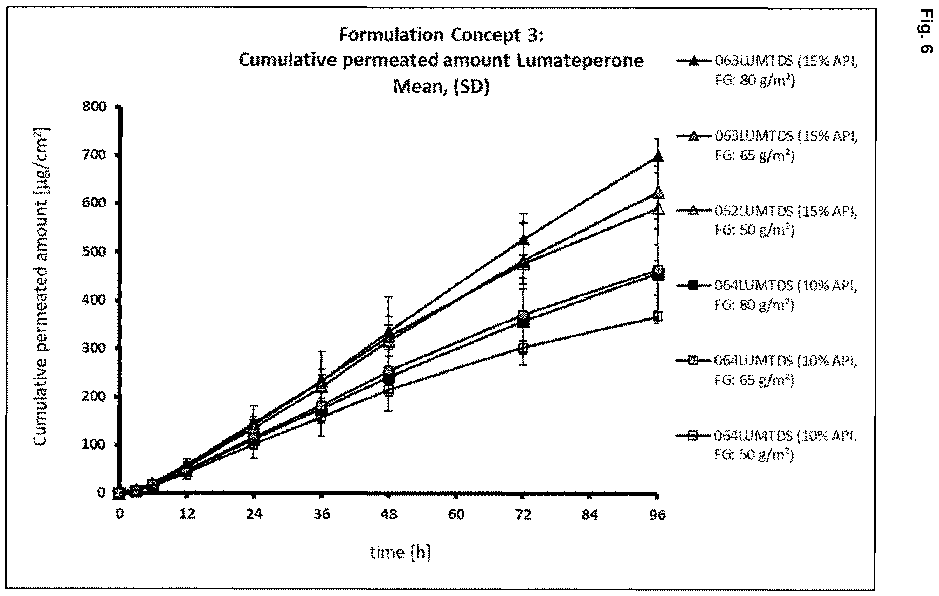

For the purpose of matrix weight optimization, selected samples with different drug concentrations and different matrix weights prepared in Example 1.2 were compared in terms of their permeation on human skin model (HSE). The results are depicted in **Fig. 6****.**

The samples show the following permeation effect on human skin model (HSE) from high to low in **Fig. 6****:**
15% Lumateperone, 85% Duro-Tak^{®} 87-6173, 80 g/m² (063LUMTDS) >
15% Lumateperone, 85% Duro-Tak^{®} 87-6173, 65 g/m² (063LUMTDS) >
15% Lumateperone, 85% Duro-Tak^{®} 87-6173, 50 g/m² (052LUMTDS) >
10% Lumateperone, 90% Duro-Tak^{®} 87-6173, 80 g/m² (064LUMTDS) >
10% Lumateperone, 90% Duro-Tak^{®} 87-6173, 65 g/m² (064LUMTDS) >
10% Lumateperone, 90% Duro-Tak^{®} 87-6173, 50 g/m²(064LUMTDS)

The dashed line in **Fig. 6** indicates the theoretically required amount of lumateperone for a 20 cm² matrix. All of the samples investigated are in accordance with this requirement, however the constant skin permeation duration varies depending onto the used drug concentration and matrix weight.

Samples with preferably 15% lumateperone and a matrix weight of 65-80 g/m² result in a high permeation and a constant permeation of about 3 days.

### g) Chemical Stability

The samples according to the following **Table 3** were prepared in accordance with the procedures described under a):

**Table 3**

| **No.** | **Batch** | **Composition of the (dried) active ingredient containing (matrix) layer** | **Matrix weight [g/m²]** | **Backing layer/ Release liner** |
|---|---|---|---|---|
| 1 | 056LUMTDS | 15% Lumateperone | 50 | Scotchpak 9738/ Primeliner 78HL |
| | | 45% Duro-Tak^{®} 87-626A, 40% Indopol H-18000 | | |
| 2 | 021LUMTDS | 15% Lumateperone | 50 | Scotchpak 9738/ Primeliner 78HL |
| | | 22.5% Duro-Tak^{®} 87-6626A, 22.5% Duro-Tak^{®} 87-6900, 20% Indopol H-1900, 20% Indopol H-18000 | | |
| | | | | |
| 3 | 057LUMTDS | 15% Lumateperone | 50 | Scotchpak 9738/ Primeliner 78HL |
| | | 85% Duro-Tak^{®} 87-6911 | | |
| 4 | 052LUMTDS | 15% Lumateperone | 50 | Scotchpak 9738/ Primeliner 78HL |
| | | 85% Duro-Tak^{®} 87-6173 | | |
| 5 | 060LUMTDS | 15% Lumateperone | 50 | Scotchpak 9738/ Primeliner 78HL |
| | | 84.95% Duro-Tak^{®} 87-6173 | | |
| | | 0.05% Tocopherol | | |
| 6 | 053LUMTDS | 15% Lumateperone | 50 | Scotchpak 9738/ Primeliner 78HL |
| | | 84.9% Duro-Tak^{®} 87-6173 | | |
| | | 0. 10% Tocopherol | | |
| 7 | 074LUMTDS | 15% Lumateperone | 50 | Scotchpak 9738/ Primeliner 78HL |
| | | 84.95% Duro-Tak^{®} 87-6173 | | |
| | | 0.05% Ascorbylpalmitate | | |
| 8 | 054LUMTDS | 15% Lumateperone | 50 | Scotchpak 9738/ Primeliner 78HL |
| | | 84.9% Duro-Tak^{®} 87-6173 | | |
| | | 0. 10% Ascorbylpalmitate | | |
| 9 | 055LUMTDS | 15% Lumateperone, 0.10% BHT (butylhydroxytolutol), 85% Duro-Tak^{®} 87-6173 | 50 | Scotchpak 9738/ Primeliner 78HL |
| | | | | |
| 10 | 017LUMTDS | 15% Lumateperone, 85% BIO-PSA 7-6302 | 50 | Scotchpak 9738/ Primeliner 78HL |

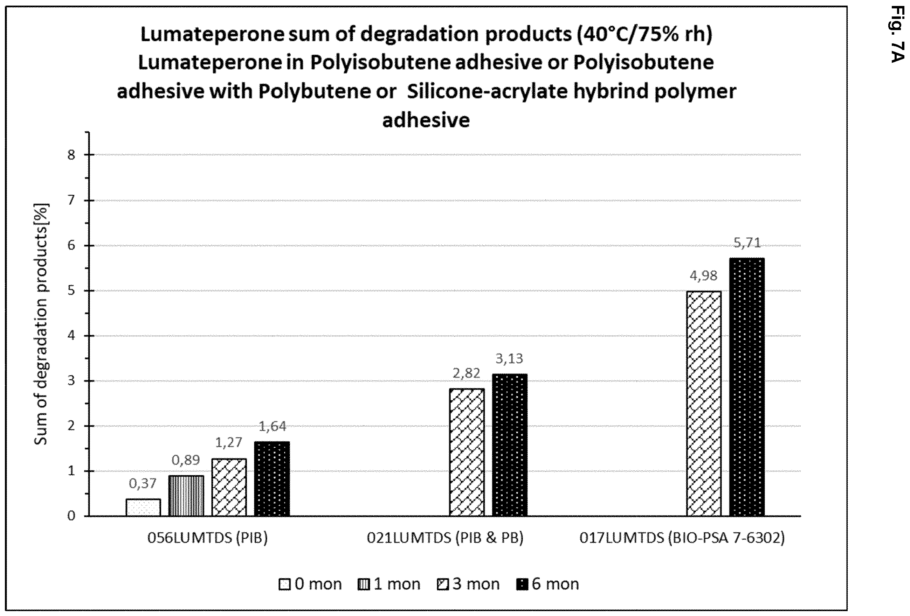

The formation of degradation products was monitored for 6 months at 40°C/75% rH. The results are shown in Fig. 7A-B.

It can be seen that, surprisingly, the use of silicone-acrylate hybrid polymer leads to a medium to high amount of degradation products, whereas the use of polyisobutene with or without polybutene leads to a medium amount of degradation products (cf. **Fig 7A**).

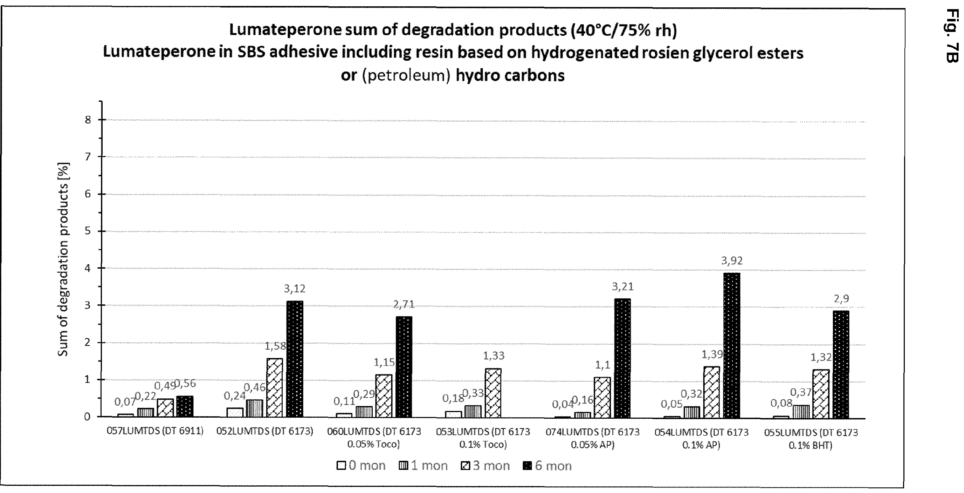

From **Fig. 7B****,** it can be seen that a polymer matrix comprising SBS and petroleum-based hydrocarbon resin leads to a relevant reduction in the formation of degradation products (cd. 057LUMTDS) and thus to a low amount of degradation products whereas a polymer matrix comprising SBS and hydrogenated rosin glycerin ester resin leads to a less relevant reduction in the formation of degradation (cd. 052LUMTDS) and thus to a medium amount of degradation products.

### Example 2: Bilayer formulation with adhesive layer (second aspect of the invention)

### a) Preparation

An active ingredient containing layer (active matrix layer) containing the active ingredient lumateperone free base, a styrene-butadiene-styrene copolymer (Duro-Tak^{®} 87-6911), and optionally, further excipients was coated on an intermediate release liner (Primeliner^{™} 78HL). The solvents were removed in a drying oven. The active ingredient containing layer was then laminated with an aluminized Scotchpak^{™} 9738 PET film as a backing layer and stored until further processing (e.g. batch 077LUMTDS).

Separately, an adhesive layer initially free of the active ingredient lumateperone free base and containing an adhesive polymer and, optionally, further excipients, was coated on a release liner (Primeliner^{™} 78HL). The solvents were removed in a drying oven.

The intermediate release liner of the active ingredient containing layer was removed and the adhesive layer initially free of active ingredient was laminated with the active matrix layer, forming a layered system consisting of: backing layer, active ingredient containing matrix layer, initially drug-free adhesive layer and release liner (protective film) (e.g. batch 077_099LUMTDS).

The resulting laminates are shown as entry No. 1 and 2 in the **Table 4.**

### Example 3: Bilayer formulation with adhesive layer and drug-permeable membrane (third aspect of the invention)

### a) Preparation

A layer containing the active ingredient lumateperone free base, a styrene-butadiene-styrene copolymer (Duro-Tak^{®} 87-6911), and, optionally, further excipients (the active ingredient containing layer after drying) was coated on an intermediate release liner (Primeliner^{™} 78HL). The solvents were removed in a drying oven. The active ingredient containing layer was then laminated with an aluminized PET film (Scotchpak^{™} 9738) as a backing layer and stored until further processing.

Separately, a layer free of the active ingredient (the adhesive layer after drying) was coated on a release liner (Primeliner^{™} 78HL). The solvents were removed in a drying oven. The adhesive layer was then laminated with a drug-permeable membrane layer.

The intermediate release liner of the active ingredient containing layer was removed and the exposed active ingredient containing layer was laminated to the membrane side of the adhesive layer, forming a layered system including: backing layer, active ingredient containing matrix layer, drug-permeable membrane, initially drug-free adhesive layer and release liner.

The resulting laminate is shown as entry No. 3 in the following **Table 4:**

**Table 4**

| **No.** | **Batch** | **Composition of the (dried) active ingredient containing (matrix) layer** | **Weight matrix [g/m²]** | **Membrane layer** |
|---|---|---|---|---|
| 1 | 077LUMTDS | 15% Lumateperone | 65 | - |
| | | 85% Duro-Tak^{®} 87-6911 | | |
| 2 | 077_099LUMTDS | 15% Lumateperone | 65 | - |
| | | 85% Duro-Tak^{®} 87-6911 | | |
| 3 | 077_100LUMTDS | 15% Lumateperone | 65 | PE |
| | | 85% Duro-Tak^{®} 87-6911 | | Cotran^{™} 9719 |

| **No.** | **Batch** | **Composition of the (dried) adhesive layer** | **Weight adhesive layer [g/m²]** | **Backing layer/ Release liner** |
|---|---|---|---|---|
| 1 | 077LUMTDS | - | - | - |
| 2 | 077_099LUMTDS | 100% Duro-Tak^{®} 87-6911 | 30 | Scotchpak 9738 Primeliner 78HL |
| 3 | 077_100LUMTDS | 100% Duro-Tak^{®} 87-6911 | 30 | Scotchpak 9738 Primeliner 78HL |

## Claims

1. Transdermal therapeutic system for administering the active ingredient lumateperone through the skin of a patient comprising the following layers:
a) a backing layer and
b) at least one active ingredient containing layer, comprising the active ingredient in free base or salt form embedded in a polymer matrix, **characterized in that**
the polymer matrix comprises a polymer selected from the group consisting of (1) a polyisobutene, (2) a styrene-butadiene-styrene copolymer, (3) a silicone-acrylate hybrid polymer, (4) a styrene-isoprene-styrene copolymer, and mixtures thereof.

2. Transdermal therapeutic system according to claim 1, comprising the active ingredient lumateperone in free base form.

3. Transdermal therapeutic system according to claim 1 or 2, **characterized in that** the active ingredient containing layer comprises lumateperone in an amount in the range from 5 to 20 wt-%, based on the overall weight of the active ingredient containing layer.

4. Transdermal therapeutic system according to any one of the preceding claims, **characterized in that** the active ingredient containing layer further comprises one or more pharmaceutically acceptable excipients selected from the group consisting of antioxidants, permeation enhancers, plasticizers, tackifiers, crystallization inhibitors and cohesion increasing substances.

5. Transdermal therapeutic system according to any one of the preceding claims, **characterized in that** the active ingredient containing layer further comprises substances that trigger the *in situ* release of lumateperone free base in case the active ingredient is used in a salt form.

6. Transdermal therapeutic system according to any one of the preceding claims, **characterized in that** the active ingredient containing layer is the skin-contacting layer of the system.

7. Transdermal therapeutic system according to any one of the preceding claims, **characterized in that** it further comprises
c) an adhesive layer on the active ingredient containing layer,
wherein the adhesive layer comprises at least one adhesive polymer,
wherein the adhesive layer is initially free of the active ingredient,
and wherein the adhesive layer is the skin-contacting layer of the system.

8. Transdermal therapeutic system according to claim 7, **characterized in that** at least one adhesive polymer of the adhesive layer is selected from the group consisting of acrylate polymers and/or copolymers, styrene-butadiene-styrene (SBS) copolymers, polyisobutylene, and a mixture of polyisobutylene and polybutene, and is preferably selected from the group consisting of a polyacrylate with free hydroxy groups and styrene-butadiene-styrene (SBS) copolymers.

9. Transdermal therapeutic system according to claim 7 or 8, **characterized in that** the adhesive layer consists of the at least one adhesive polymer or further comprises one or more pharmaceutically acceptable excipients selected from the group consisting of enhancers, plasticizers, cohesion increasing substances, antioxidants.

10. Transdermal therapeutic system according to any one of claims 7 to 9, **characterized in that** it further comprising
d) a membrane layer between the active ingredient containing layer and the adhesive layer,
wherein the membrane layer comprises a membrane polymer that is permeable for the active ingredient and controls the release of the active ingredient.

11. Transdermal therapeutic system according to claim 10, **characterized in that** the membrane polymer is a polyolefin selected from the group consisting of polypropylene, polyethylene, and of polyethylene vinyl acetate.

12. Transdermal therapeutic system according to any one of the preceding claims, **characterized in that** it further comprises
e) a release liner to protect the skin-contacting layer before use of the system.

13. Transdermal therapeutic system according to any one of the preceding claims, **characterized in that** the permeation is constant over a period of at least 48 hours, preferably at least 72 hours; and/or the flux is at least 3.75 µg/cm²*h; and/or the daily dosage delivered is at least 1.5 mg/day, preferably 1.8 mg/day; and/or the size of the transdermal therapeutic system is in the range from 4 to 40 cm².

14. Transdermal therapeutic system according to any one of the preceding claims, **characterized in that** it does not comprise a permeation enhancer.

15. A method for preparing a transdermal therapeutic system according to any one of the preceding claims, comprising the following steps:
(a) preparing an active ingredient containing layer composition,
(b) coating the composition prepared in step (a) onto a release liner (i),
(c) drying the component prepared in step (b), and
(d) laminating a backing layer onto the active ingredient layer of the component prepared in step (c);
optionally comprising the following steps in addition to steps (a)-(d):
(e) preparing an adhesive layer composition,
(f) coating the composition prepared in step (e) onto a release liner (ii),
(g) drying the component prepared in step (f),
(h) removing the release liner (i) from the component prepared in step (d),
(i) laminating the active ingredient containing layer of the component prepared in step (h) onto the adhesive layer of the component prepared in step (g), or *vice versa;*
or optionally comprising the following steps in addition to steps (a)-(d):
(j) laminating the membrane layer suitable to control the release of the active ingredient onto the active ingredient containing layer of the component prepared in accordance with step (h),
(k) performing steps (e) to (g),
(l) laminating the adhesive layer of the component prepared in step (k) onto the membrane layer of the component prepared in step (j), or *vice versa.*

16. Use of a polymer selected from the group consisting of (1) a polyisobutene, (2) a styrene-butadiene-styrene copolymer, (3) a silicone-acrylate hybrid polymer, (4) a styrene-isoprene-styrene copolymer, and mixtures thereof for stabilizing lumateperone free base in a transdermal therapeutic system, or reducing decomposition of lumateperone free base in a transdermal therapeutic system, preferably in a transdermal therapeutic system as defined in any one of claims 1 to 14.

17. Use of an antioxidant, preferably ascorbic acid or tocopherol, in a transdermal therapeutic system as defined in any one of claims 1 to 14 for reducing, preferably inhibiting the formation of N-nitrosamines in the system.

18. Transdermal therapeutic system according to any once of claims 1 to 14 for use in a method for treating a disease selected from the group consisting of major depressive disorder (MDD) and adjunctive mixed features associated therewith, bipolar depression I or II and schizophrenia.

19. Lumateperone for use in a method for treating a disease selected from the group consisting of major depressive disorder (MDD) and adjunctive mixed features associated therewith, bipolar depression I or II and schizophrenia, wherein lumateperone is administered in free base or salt form through the skin of a patient by means of the transdermal therapeutic system as defined in any one of claims 1 to 14.
